(19) **Europäisches Patentamt European Patent Office Office européen des brevets**

(11) **EP 3 174 978 B1**

(12) **FASCICULE DE BREVET EUROPEEN**

(45) Date de publication et mention de la délivrance du brevet:
**11.09.2019 Bulletin 2019/37**

(51) Int Cl.:
*C12N 9/10* *(2006.01)*    *C12P 19/08* *(2006.01)*
*C12P 19/18* *(2006.01)*   *A61K 8/73* *(2006.01)*
*A61K 47/36* *(2006.01)*   *A61K 31/721* *(2006.01)*
*C08B 37/02* *(2006.01)*   *A23L 29/269* *(2016.01)*

(21) Numéro de dépôt: **15759857.4**

(22) Date de dépôt: **21.07.2015**

(86) Numéro de dépôt international:
**PCT/FR2015/052002**

(87) Numéro de publication internationale:
**WO 2016/016544 (04.02.2016 Gazette 2016/05)**

(54) **PROTEINE A ACTIVITE DEXTRANE-SACCHARASE ET APPLICATIONS**

PROTEIN MIT DEXTRANSACCHARASEAKTIVITÄT UND VERWENDUNGEN

PROTEIN WITH DEXTRAN-SACCHARASE ACTIVITY, AND USES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.07.2014 FR 1457264**

(43) Date de publication de la demande:
**07.06.2017 Bulletin 2017/23**

(73) Titulaires:
- **Institut National des Sciences Appliquées de Toulouse**
  **31077 Toulouse Cedex 4 (FR)**
- **Institut National De La Recherche Agronomique**
  **75007 Paris (FR)**
- **Centre National de la Recherche Scientifique**
  **75016 Paris (FR)**
- **Université Paul Sabatier Toulouse III**
  **31400 Toulouse (FR)**

(72) Inventeurs:
- **VUILLEMIN, Marlène**
  **2900 Hellerup (DK)**
- **CLAVERIE, Marion**
  **F-64800 Benejacq (FR)**
- **MOULIS, Claire**
  **F-31290 Viellevigne (FR)**
- **REMAUD-SIMEON, Magali**
  **F-31520 Ramonville-Saint-Agne (FR)**
- **MONSAN, Pierre**
  **F-31700 Mondonville (FR)**
- **SEVERAC, Etienne**
  **F-31820 Pibrac (FR)**
- **FONTAGNE-FAUCHER, Catherine**
  **F-32600 L'Isle Jourdain (FR)**

(74) Mandataire: **Cabinet Plasseraud**
  **66, rue de la Chaussée d'Antin**
  **75440 Paris Cedex 09 (FR)**

(56) Documents cités:
**EP-A2- 0 164 656**      **WO-A1-2010/129839**
**WO-A2-2010/128859**    **US-A- 4 767 614**
**US-A- 5 229 277**

- **DOLS M ET AL: "Characterization of the different dextransucrase activities excreted in glucose, fructose, or sucrose medium by Leuconostoc mesenteroides NRRL B-1299", APPLIED AND ENVIRONMENTAL MICROBIOLOGY, AMERICAN SOCIETY FOR MICROBIOLOGY, US, vol. 64, no. 4, avril 1998 (1998-04), pages 1298-1302, XP002360116, ISSN: 0099-2240**
- **SIMPSON C ET AL: "Four glucosyltransferases, GtfJ, GtfK, GtfL, and GtfM from Streptococcus salivarius ATCC 25975", MICROBIOLOGY AND IMMUNOLOGY, CENTER FOR ACADEMIC PUBLICATIONS JAPAN|, JP, vol. 141, no. 6, juin 1995 (1995-06), pages 1451-1460, XP002082272, ISSN: 0385-5600**

- Anonymous: "Dextrans", , 20 janvier 2014 (2014-01-20), XP055218107, Extrait de l'Internet: URL:https://web.archive.org/web/2014012004 0848/http://www.sigmaaldrich.com/life-scie nce/biochemicals/biochemical-products.html ? TablePage=22696471 [extrait le 2015-10-05]
- Vicki Caligur: "Dextran and Related Polysaccharides", BioFiles, 2008, XP055218106, Extrait de l'Internet: URL:http://www.sigmaaldrich.com/content/da m/sigma-aldrich/articles/biofiles/volume3. 10a17/Dextran-and-Related-Polysaccharides. pdf [extrait le 2015-10-05]
- ELVIRA KHALIKOVA ET AL: "Microbial Dextran-Hydrolyzing Enzymes: Fundamentals and Applications", MICROBIOLOGY AND MOLECULAR BIOLOGY REVIEWS, vol. 69, no. 2, juin 2005 (2005-06), pages 306-325306, XP055218315, DOI: 10.1128/JMBR.69.2.306-325.2005
- S. A. F. T. VAN HIJUM ET AL: "Structure-Function Relationships of Glucansucrase and Fructansucrase Enzymes from Lactic Acid Bacteria", MICROBIOLOGY AND MOLECULAR BIOLOGY REVIEWS, vol. 70, no. 1, mars 2006 (2006-03), pages 157-176, XP055179067, ISSN: 1092-2172, DOI: 10.1128/MMBR.70.1.157-176.2006
- DATABASE UniProt [Online] 4 février 2015 (2015-02-04), "SubName: Full=Alternansucrase {ECO:0000313|EMBL:CDX66895.1}; EC=2.4.1.140 {ECO:0000313|EMBL:CDX66895.1};", XP002745403, extrait de EBI accession no. UNIPROT:A0A0A1IPZ8 Database accession no. A0A0A1IPZ8

Remarques:

Le dossier contient des informations techniques présentées postérieurement au dépôt de la demande et ne figurant pas dans le présent fascicule.

**Description**

L'invention a pour objet une protéine à activité dextrane-saccharase issue de la souche *Leuconostoc citreum* NRRL B-1299, notamment une protéine à activité dextrane-saccharase tronquée. L'invention concerne également un procédé de synthèse de dextranes au moyen d'une telle protéine à activité dextrane-saccharase, ainsi que les dextranes synthétisés.

Par la suite, pour plus de clarté le terme « dextrane-saccharase » pourra parfois être utilisé pour désigner une protéine à activité dextrane-saccharase.

Les glucane-saccharases d'origine bactérienne sont des $\alpha$-transglucosylases appartenant aux familles 13 et 70 des glycoside-hydrolases. A partir de saccharose, ces enzymes catalysent généralement la synthèse d'$\alpha$-glucanes de haute masse molaire ($10^6$-$10^9$ g.mol$^{-1}$). Elles peuvent également synthétiser des oligosaccharides ou glucoconjugués par réaction de transglucosylation sur des accepteurs exogènes de natures variées. Les glucanes-saccharases présentent des spécificités de produits différentes, tant au niveau de la nature des liaisons osidiques synthétisées ($\alpha$-1,2 ; $\alpha$-1,3 ; $\alpha$-1,4 ou $\alpha$-1,6) et leur organisation, que de la taille des produits formés.

De manière générale, les glucanes-saccharases sont naturellement produites par des bactéries lactiques, par exemple des genres *Leuconostoc*, *Lactobacillus*, *Streptococcus* ou *Weissela* sp.

Parmi les glucane-saccharases, les dextrane-saccharases produisent du dextrane, présentant de manière générale au moins 50% de liaisons osidiques $\alpha$-1,6 dans la chaîne principale, et éventuellement des ramifications en $\alpha$-1,2, $\alpha$-1,3 et/ou $\alpha$-1,4. Le taux de ramifications ainsi que leur arrangement spatial varient suivant l'enzyme productrice.

Les dextranes et les dérivés de dextranes ont un nombre d'applications industrielles croissant, souvent dépendant de leurs masses molaires.

Sauf indication contraire, on entend par « *masse molaire* » ou « *masse molaire moyenne* », dans la présente invention, la masse molaire moyenne en poids, exprimée indépendamment en g.mol$^{-1}$ ou Da.

Les dextranes de masse molaire faible ou moyenne (allant généralement de $10^3$ à $7.10^4$ g.mol$^{-1}$) sont notamment utilisés pour des applications analytiques, dans le domaine médical, par exemple en tant qu'extenseur du plasma sanguin grâce à leur faible caractère antigénique et leur faible viscosité en solution saline, pour des solutions ophtalmiques, transporteur de fer ou anticoagulant (après fonctionnalisation), dans la prévention des chocs post-opératoires, dans le traitement des brûlures ou dans la réduction de risques de thrombose ou d'embolies, etc.

Ces dextranes de masse molaire faible ou moyenne sont en général produits par hydrolyse acide, suivi d'un fractionnement à l'aide de solvants organiques. Cependant, ces procédés chimiques sont souvent coûteux, peu rentables et polluants.

Des procédés alternatifs ont donc été développés afin d'améliorer la production de dextranes de masse molaire faible ou moyenne.

Ainsi, le brevet US 5,229,277 décrit un procédé de synthèse de dextranes de faible masse molaire en mettant en oeuvre l'utilisation de *Leuconostoc mesenteroides* et une souche mutante de *Lipomyces starkeyi* ATCC 74054. Ce procédé, en plus de requérir l'utilisation de deux microorganismes, nécessite des conditions de culture particulières et des durées et températures précises pour que l'activité dextranase identifiée chez *Lipomyces starkeyi* ATCC 74054 réduise la masse molaire des dextranes synthétisés par *Leuconostoc mesenteroides.* Les polymères de dextrane produits ont une masse molaire comprise entre $4.10^4$ et $1,5.10^5$ Da.

La demande de brevet EP2365084 décrit un procédé de synthèse de dextranes de masse molaire contrôlée. Ces $\alpha$-glucanes sont produits directement par des formes tronquées de la glucane-saccharases DsrS, issue de *Leuconostoc mesenteroides* NRRL B-512 F, à partir de saccharose, avec l'ajout optionnel d'un accepteur exogène. Un variant DsrS Core $\Delta$A est particulièrement intéressant pour la production de dextrane de masse molaire moyenne de $1.10^4$ Da.

Cependant la synthèse doit se faire à des températures faibles (préférentiellement à 10 °C) afin de maximiser les rendements. De plus l'enzyme ne présente pas une grande efficacité catalytique, et environ 48 heures de synthèse sont nécessaires pour atteindre la consommation totale du saccharose.

Il est aussi possible de favoriser la production de dextranes de faible masse molaire en augmentant la concentration initiale en substrat. Cependant, il a été souvent démontré avec cette méthode que le dextrane produit était bien souvent polydisperse, et que la synthèse de polymères de haute masse molaire n'était pas complètement annihilée.

Il existe donc toujours un besoin pour la production de dextranes ayant une faible masse molaire, notamment des dextranes peu polydisperses.

Un objectif de l'invention était également de fournir des dextranes linéaires. De tels dextranes sont en effet utiles dans de nombreux domaines, notamment pour des applications médicales. En effet, il a été montré que les dextranes présentant une forte teneur en liaisons $\alpha$-1,6 (et donc les plus linéaires) sont ceux qui entrainent le moins de réactions allergènes (1, 2).

Un autre objectif de l'invention était également de fournir un procédé de production de tels dextranes de manière enzymatique, et non chimique, et de manière simple, rapide et précise, ne nécessitant l'utilisation que d'un unique microorganisme.

Un autre objectif de l'invention était de pouvoir maîtriser la masse molaire d'un dextrane synthétisé de manière très précise, notamment au moyen de paramètre(s) de réaction aisément contrôlable(s).

Les inventeurs ont ainsi le mérite d'avoir découvert qu'une protéine à activité dextrane-saccharase issue de la souche *Leuconostoc citreum* NRRL B-1299 permet la synthèse de dextranes de masse molaire contrôlable de manière très précise, et ceci, sans nécessiter de réactions enzymatiques contraignantes, directement à partir de saccharose et sans ajout d'accepteur exogène ou d'autre(s) enzyme(s).

Une telle protéine a pour séquence d'acides aminés la séquence SEQ ID NO : 1. La protéine ne possède qu'un maximum d'identité de 62% sur 100% de sa séquence avec l'alternane-saccharase de *Leuconostoc mesenteroides* LBAE C11 dont la séquence est disponible dans les bases de données sous le numéro d'accession Genbank WP_004904957.1. Comparé à d'autres séquences putatives de glucane-saccharases pouvant être identifiées suite à des campagnes de séquençage de génomes bactériens, ce pourcentage d'identité est plutôt faible.

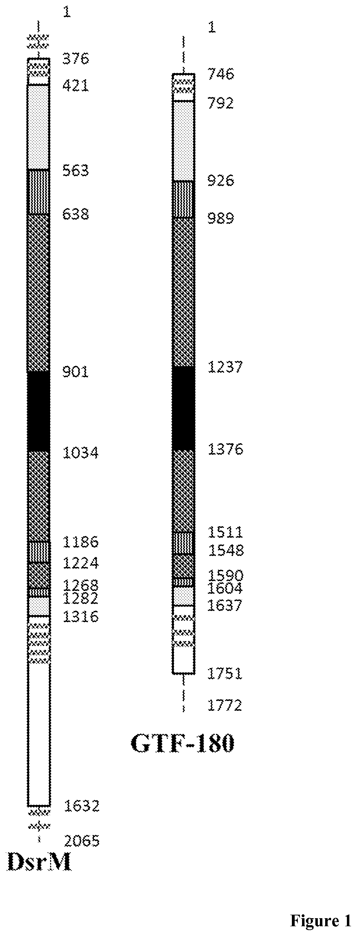

La protéine à activité dextrane-saccharase de séquence d'acides aminés SEQ ID NO : 1, appelée DsrM, de masse molaire 229 kDa, est composée de 2065 acides aminés, possédant la triade catalytique DED et les 4 motifs conservés décrits habituellement chez les enzymes de la famille 70 des glycoside-hydrolases. Les motifs protéiques conservés du coeur catalytique (I à IV) ont ainsi été identifiés de la position 1177 à la position 1183 pour le motif I, de la position 673 à la position 683 pour le motif II, de la position 710 à la position 721 pour le motif III et de la position 785 à la position 799 pour le motif IV. En comparaison avec la séquence protéique de la glucane-saccharase GTF-180, les cinq domaines structuraux classiquement décrits pour les glucane-saccharases (A, B, C, IV et V) (3) peuvent être identifiés dans la structure primaire de la protéine à activité dextrane-saccharase de séquence d'acides aminés SEQ ID NO : 1 (figure 1).

Les variants conservateurs de fonction de la protéine à activité dextrane-saccharase de séquence d'acides aminés SEQ ID NO : 1 sont également compris dans la présente demande.

On appelle « *variant conservateur de fonction* », un variant dans lequel un ou plusieurs résidu(s) d'acide aminé donné(s) dans une protéine a (ont) été modifié(s) ou supprimé(s) ou inséré(s) sans pour autant altérer la conformation globale et l'activité enzymatique de la protéine à activité dextrane-saccharase. L'activité enzymatique d'un variant de dextrane-saccharase peut être testée selon des techniques connues de l'homme du métier, par exemple par la méthode à l'acide dinitrosalicylique (DNS) de Sumner et Howell (4) ou par analyse HPLC.

De préférence, un tel variant conservateur de fonction est une protéine à activité dextrane-saccharase dont la séquence d'acides aminés a au moins 80%, de préférence 85%, de préférence encore 90%, de préférence encore 95%, de préférence encore 98% d'identité sur la séquence d'acides aminés SEQ ID NO : 1.

De manière avantageuse, la ou les modification(s) concernent des parties non sensibles de l'enzyme, et ne concernent donc notamment pas la zone allant de la position 563 à la position 1282 de la séquence d'acides aminés SEQ ID NO : 1, correspondant à la triade catalytique et aux domaines A, B, C.

De préférence, la ou les modifications ne concernent notamment pas la zone allant de la position 563 à la position 1282 de la séquence d'acides aminés SEQ ID NO : 1 ainsi que les zones allant de la position 1316 à 1433 (partie du domaine V en C-terminal) de la séquence d'acides aminés SEQ ID NO : 1.

De préférence encore, la ou les modifications ne concernent notamment pas la zone allant de la position 563 à la position 1282 de la séquence d'acides aminés SEQ ID NO : 1, les zones allant de la position 1316 à 1433 de la séquence d'acides aminés SEQ ID NO : 1 ainsi que les zones allant de la position 174 à 421 de la séquence d'acides aminés SEQ ID NO : 1.

Les séquences allant des positions 174 à 421, puis de 1316 à 1433 comportent en effet des unités répétées (YG repeats) généralement décrites dans cette famille pour avoir une affinité pour le glucane et donc jouer un rôle sur la taille des produits formés, ainsi que sur l'activité catalytique de l'enzyme (3).

De préférence encore, la ou les modifications ne concernent notamment pas la zone allant de la position 563 à la position 1282 de la séquence d'acides aminés SEQ ID NO : 1, les zones allant de la position 1316 à 1433 de la séquence d'acides aminés SEQ ID NO : 1 ainsi que les zones allant de la position 42 à 421 de la séquence d'acides aminés SEQ ID NO : 1.

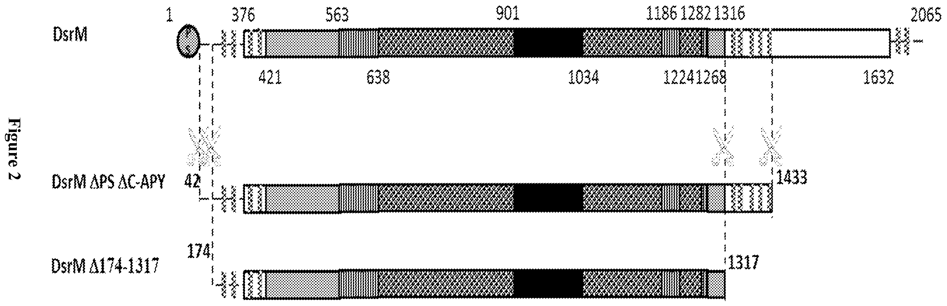

Ainsi, il a été mis en évidence lors d'essais de troncature de la dextrane-saccharase de séquence d'acides aminés SEQ ID NO : 1 que la suppression des acides aminés de la position 1434 à 2065 du domaine V à l'extrémité C-terminale, dont notamment la totalité des « motifs APY », n'impactait pas l'activité et la spécificité de l'enzyme (cf. figure 2, dextrane-saccharase tronquée DsrM ΔPS ΔC-APY).

En revanche, il a été mis en évidence que la suppression des acides aminés de la position 1 à 174 et de la position 1317 à 2065 de la séquence d'acides aminés SEQ ID NO : 1 (domaine V à l'extrémité C-terminale), faisait perdre à l'enzyme son activité (cf. figure 2, dextrane-saccharase tronquée DsrM Δ174-1317).

L'homme du métier est capable de déterminer les variants conservateurs de fonction, notamment au regard de ces éléments.

Ainsi, un objet de l'invention est une protéine à activité dextrane-saccharase ayant pour séquence d'acides aminés la séquence SEQ ID NO : 1, ou comprenant au moins 80%, de préférence 85%, de préférence encore 90%, de préférence encore 95%, de préférence encore 98% d'identité sur les positions 563 à 1282 de la séquence d'acides aminés SEQ ID NO : 1, de préférence sur les positions 563 à 1282 et 1316 à 1433 de la séquence d'acides aminés SEQ ID NO : 1, de préférence encore sur les positions 563 à 1282, 1316 à 1433 et 174 à 421 de la séquence d'acides aminés SEQ ID NO : 1, de préférence encore sur les positions 563 à 1282, 1316 à 1433 et 42 à 421 de la séquence d'acides aminés SEQ ID NO : 1.

Selon un mode de réalisation particulier, un variant conservateur de fonction consiste en une forme tronquée d'une protéine à activité dextrane-saccharase telle que définie précédemment et qui conserve l'activité enzymatique dextrane-saccharase.

De préférence, une telle forme tronquée comprend moins de 2065 acides aminés, de préférence moins de 1600 acides aminés, de préférence encore moins de 1450 acides aminés. Très préférentiellement, une telle forme tronquée comprend 1392 acides aminés.

Selon un mode de réalisation particulier, une telle forme tronquée de la protéine à activité dextrane-saccharase de séquences d'acides aminés SEQ ID NO : 1 a pour séquence d'acides aminés la séquence SEQ ID NO : 2. Cette forme tronquée particulière est appelée DsrM ΔPS ΔC-APY.

La séquence d'acides aminés SEQ ID NO : 2 est identique à la séquence d'acides aminés SEQ ID NO : 1 de la position 42 à la position 1433, et a été délétée des positions 1 à 41 et 1434 à 2065 de la séquence d'acides aminés SEQ ID NO : 1. Comme mentionné précédemment, il a ainsi été mis en évidence que la suppression de ces acides aminés, dont notamment la totalité des « motifs APY », n'impacte pas l'activité et la spécificité de l'enzyme.

Par ailleurs, la protéine à activité dextrane-saccharase de séquence d'acides aminés SEQ ID NO : 2 ne dépasse pas 54% d'identité, et 68% de similarité, avec les séquences protéiques de DsrS et ses variants DsrS vardel Δ3, DsrS vardel Core et DsrS Core ΔA (cf. la demande de brevet EP2365084).

Les variants conservateurs de fonction de la protéine à activité dextrane-saccharase de séquence d'acides aminés SEQ ID NO : 2 sont également compris dans la présente demande et sont définis de la même façon que précédemment.

Ainsi, de préférence, la ou les modifications peuvent concerner des parties non sensibles de l'enzyme, et ne concernent donc notamment pas la zone allant de la position 522 à la position 1241 de la séquence d'acides aminés SEQ ID NO: 2, correspondant à la triade catalytique et aux domaines A, B, C.

De préférence, la ou les modifications ne concernent notamment pas la zone allant de la position 522 à la position 1241 de la séquence d'acides aminés SEQ ID NO : 2 ainsi que les zones allant de la position 1275 à 1392 (partie du domaine V en C-terminal) de la séquence d'acides aminés SEQ ID NO : 2.

De préférence encore, la ou les modifications ne concernent notamment pas la zone allant de la position 522 à la position 1241 de la séquence d'acides aminés SEQ ID NO : 2, les zones allant de la position 1275 à 1392 de la séquence d'acides aminés SEQ ID NO : 2 ainsi que les zones allant de la position 133 à 380 de la séquence d'acides aminés SEQ ID NO : 2.

De préférence encore, la ou les modifications ne concernent notamment pas la zone allant de la position 522 à la position 1241 de la séquence d'acides aminés SEQ ID NO : 2, les zones allant de la position 1275 à 1392 de la séquence d'acides aminés SEQ ID NO : 2 ainsi que les zones allant de la position 1 à 380 de la séquence d'acides aminés SEQ ID NO : 2.

Ainsi, un objet de l'invention concerne une protéine à activité dextrane-saccharase ayant pour séquence d'acides aminés la séquence SEQ ID NO : 2, ou comprenant au moins 80%, de préférence 85%, de préférence encore 90%, de préférence encore 95%, de préférence encore 98% d'identité sur les positions 522 à 1241 sur la séquence d'acides aminés SEQ ID NO : 2, de préférence sur les positions 522 à 1241 et 1275 à 1392 sur la séquence d'acides aminés SEQ ID NO : 2, de préférence encore sur les positions 522 à 1241, 1275 à 1392 et 133 à 380 sur la séquence d'acides aminés SEQ ID NO : 2, de préférence encore sur les positions 522 à 1241, 1275 à 1392 et 1 à 380 sur la séquence d'acides aminés SEQ ID NO : 2.

Par la suite, le terme «protéine à activité dextrane-saccharase de séquence d'acides aminés SEQ ID NO: 2 » (ou «dextrane-saccharase de séquence d'acides aminés SEQ ID NO : 2 ») se rapporte aussi bien à la protéine à activité dextrane-saccharase de séquence d'acides aminés SEQ ID NO : 2 qu'à ses variants conservateurs de fonction. Selon un mode de réalisation particulier, il s'agit uniquement de la protéine à activité dextrane-saccharase de séquence d'acides aminés SEQ ID NO : 2.

Les propriétés fonctionnelles exceptionnelles d'une dextrane-saccharase selon l'invention ont été mises en évidence par les inventeurs.

Une protéine à activité dextrane-saccharase selon l'invention est exclusivement spécifique de la polymérisation par liaisons osidiques de type α-1,6, et de surcoût est une excellente polymérase. En effet, comme mis en évidence dans la partie expérimentale, les analyses chromatographiques réalisées suite à une synthèse de dextrane à partir de 100 g.L$^{-1}$ de saccharose montrent qu'environ 85% et 81% des unités glucosyle issues du saccharose sont utilisées pour la production du polymère lors de l'utilisation respective d'une dextrane-saccharase de séquence d'acides aminés SEQ ID NO : 2 et d'une dextrane-saccharase de séquence d'acides aminés SEQ ID NO : 1.

En outre, une protéine à activité dextrane-saccharase selon l'invention a une température optimale de fonctionnement comprise entre 30 °C et 45 °C, et a un pH optimal compris entre 4 et 7, plus précisément entre 4,5 et 5,75 pour une protéine à activité dextrane-saccharase de séquence d'acides aminés SEQ ID NO : 2, et entre 5 et 7 pour une protéine à activité dextrane-saccharase de séquence d'acides aminés SEQ ID NO : 1.

Une dextrane-saccharase selon l'invention offre ainsi une large gamme d'utilisation en termes de pH et de températures. Notamment, l'enzyme conserve plus de 80% de son activité sur une large gamme de températures, notamment jusqu'à 45 °C, ce qui est très rare chez les enzymes de la famille 70 des glycoside-hydrolases. Or, la mise en oeuvre de la synthèse de dextranes à des températures élevées, par exemple environ 45 °C, permet de limiter certaines contaminations microbiennes. Cette large gamme d'utilisation rend par conséquent la dextrane-saccharase selon l'invention très attractive pour des utilisations industrielles.

Selon un mode de réalisation particulier, une protéine à activité dextrane-saccharase selon l'invention peut être préparée par des techniques connues de recombinaison génétique.

L'homme du métier saura utiliser les technologies de biologie moléculaire et saura choisir un système d'expression adapté selon les techniques qui lui sont connues. On peut ici se référer aux résultats expérimentaux ci-après.

Le terme "*système d'expression*" comprend une cellule hôte et un vecteur compatible dans des conditions appropriées, c'est-à-dire des conditions permettant l'expression du gène codant pour la protéine d'intérêt, porté par le vecteur et introduit dans la cellule hôte. Typiquement, la séquence d'acides nucléiques codant une protéine à activité dextrane-saccharase selon l'invention, peut être insérée dans un vecteur d'expression approprié qui sera alors introduit dans une cellule hôte procaryote ou eucaryote adéquate.

Tout vecteur d'expression approprié et connu de l'homme du métier peut être utilisé selon l'invention. Un vecteur d'expression est typiquement un plasmide, un cosmide, un épisome, un chromosome artificiel, un phage ou un vecteur viral. De manière avantageuse, le vecteur d'expression utilisé est pET-53-DEST, pET-55-DEST, pET-60-DEST ou pBAD-DEST49.

Typiquement, l'expression des acides nucléiques de la présente invention peut être réalisée dans des cellules hôtes procaryotes ou eucaryotes. Comme exemples non limitatifs de souches de cellule hôte procaryotes, on peut citer des souches telles que *Escherichia coli, Bacillus subtilis, Salmonella typhimurium* ou des souches du genre *Pseudomonas, Streptomyces* et *Staphylococcus.* Comme exemples non limitatifs de souches de cellules hôtes eucaryotes, on peut citer des souches telles que des parasites *Apicomplexan* (*Plasmodia*, *Toxoplasma, Cryptosporidia) Leishmania* ou *Trypanosoma*, ou des cellules de levure telles que *Saccharomyces sp.* comme *Saccharomyces cerevisiae* ou *pombe*, *Pichia pastoris* etc.

De manière préférentielle, des cellules hôtes procaryotes sont utilisées. Selon un mode de réalisation avantageux, les cellules utilisées pour l'expression des acides nucléiques de la présente invention sont *Escherichia coli* et de manière encore préférentielle, les souches sont choisies parmi TOP10, BL21-AI, BL21 Star DE3, Arctic Express DE3.

Selon un mode de réalisation particulier dans lequel la dextrane-saccharase selon l'invention est une dextrane-saccharase tronquée telle que précédemment définie, la séquence d'acides nucléiques codant une dextrane-saccharase de séquence d'acides aminés SEQ ID NO : 2 telle que précédemment décrite, est insérée dans un vecteur d'expression pET-55-DEST, alors introduit dans une cellule hôte procaryote de type *Escherichia coli* BL21 Star DE3. Ce mode de réalisation permet de produire la dextrane-saccharase, et permet d'obtenir environ 10 000 Unités d'activité enzymatique par litre de culture.

Une dextrane-saccharase selon l'invention peut être préparée par culture de cellules hôtes contenant une séquence d'acide nucléique codant une dextrane-saccharase selon l'invention, dans des conditions permettant l'expression d'une dextrane-saccharase, et par isolement de ladite dextrane-saccharase du milieu de culture selon les techniques connues de l'homme du métier.

De telles dextrane-saccharases peuvent ensuite être purifiées par toute technique de purification connue de l'homme du métier, par exemple par précipitation, chromatographie par échange d'ions, chromatographie d'affinité, chromatographie d'échange hydrophobe, filtration sur gel, chromatographie HPLC en phase inverse, etc. Selon un mode de réalisation préférentiel, la dextrane-saccharase obtenue par culture de cellules hôtes est purifiée par chromatographie d'affinité.

De manière avantageuse, une dextrane-saccharase selon l'invention peut également être immobilisée par des techniques connues de l'homme du métier, par exemple, par formation de liaisons ioniques et/ou hydrophobes entre le support et la protéine (adsorption), formation de liaisons covalentes entre le support et la protéine, inclusion de la protéine dans le support ou encapsulation de la protéine dans le support. L'immobilisation d'une dextrane-saccharase selon l'invention constitue un mode de réalisation particulièrement avantageux selon l'invention, comme il sera vu ci-après.

En raison de leurs propriétés dextrane-saccharase remarquables, les protéines à activité dextrane-saccharase selon l'invention permettent en outre la synthèse de dextranes. L'homme du métier saura adapter les conditions expérimentales à appliquer pour avoir une production optimisée de dextrane. D'une manière générale, la dextrane-saccharase doit être incubée dans un milieu de synthèse contenant du saccharose.

Par conséquent, un objet de l'invention se rapporte à un procédé de synthèse de dextranes, dans lequel :

- on fournit du saccharose dans un milieu de synthèse,
- on met en contact une dextrane-saccharase selon l'invention avec ledit saccharose dans ledit milieu de synthèse pour former des dextranes,
- on isole optionnellement les dextranes obtenus.

De manière avantageuse, le pH utilisé pour le procédé de synthèse est compris entre 4 et 7, de préférence entre 4,5 et 5,75 pour une dextrane-saccharase de séquence d'acides aminés SEQ ID NO : 2, et entre 5 et 7 pour une dextrane-saccharase de séquence d'acides aminés SEQ ID NO : 1. Typiquement, le pH utilisé pour le procédé de fabrication est d'environ 5,75.

Le milieu de synthèse peut être tout milieu connu de l'homme du métier et adapté à la synthèse de dextranes selon l'invention. De préférence, le milieu de synthèse est un milieu liquide comprenant un solvant. L'homme pourra notamment utiliser tout solvant permettant d'atteindre le pH souhaité pour la synthèse. Ainsi, ledit solvant pourra, de manière illustrative, être choisi parmi l'eau, ou une solution tampon appropriée (par exemple l'acétate de sodium).

L'homme du métier saura adapter la durée de la synthèse, en particulier en fonction de la quantité de protéine à activité dextrane-saccharase ajoutée dans le milieu de synthèse et en fonction de la température. Ainsi, une durée de synthèse plus longue sera nécessaire lorsque de fortes concentrations de saccharose dans le milieu de synthèse seront utilisées, par exemple de l'ordre de 400 g.L$^{-1}$ à 600 g.L$^{-1}$. Typiquement, à une concentration enzymatique de 1 U.mL$^{-1}$, la synthèse est conduite pendant une durée comprise entre 4 heures et 24 heures, de préférence comprise entre 8 heures et 16 heures, de préférence pendant une durée d'environ 14 heures.

De manière particulièrement avantageuse, de larges gammes de température et de concentrations initiales en substrat peuvent être utilisées, celles-ci permettant en outre le contrôle précis de la masse molaire des dextranes obtenus.

De manière générale, la concentration en saccharose dans le milieu de synthèse peut être comprise entre 50 et 600 g.L$^{-1}$, de préférence entre 50 et 400 g.L$^{-1}$. Typiquement, on utilisera des concentrations en saccharose dans le milieu de synthèse entre 50 et 200 g.L$^{-1}$ pour des dextranes à masse molaire plus élevée, et on utilisera des concentrations en saccharose dans le milieu de synthèse entre 200 et 600 g.L$^{-1}$, de préférence entre 200 et 400 g.L$^{-1}$ pour des dextranes à masse molaire plus faible. Il est en effet déjà connu que la masse molaire moyenne du dextrane synthétisé sera susceptible d'être plus élevée en utilisant une concentration initiale en saccharose faible dans le milieu de synthèse.

Par ailleurs, de manière générale, la synthèse peut être conduite à une température comprise entre 20 °C et 50 °C, de préférence comprise entre 25 °C et 45 °C. Comme mentionné précédemment, une utilisation de l'enzyme sur une large gamme de températures, notamment jusqu'à 45 °C, est très rare chez les enzymes de la famille 70 des glycoside-hydrolases. Or, la mise en oeuvre de la synthèse de dextranes à des températures élevées, par exemple environ 45 °C, permet de limiter certaines contaminations microbiennes.

Par ailleurs, il a été montré dans de précédents travaux qu'une augmentation de la température permettait l'obtention de dextranes de masses molaires plus élevées (5,6). Or, il a ici été mis en évidence de manière surprenante que, au contraire, lors de la synthèse de dextranes au moyen d'une dextrane-saccharase selon l'invention, une diminution de la température induisait des dextranes de masses molaires plus élevées.

Typiquement, on utilisera des températures entre 20 °C et 35 °C, de préférence entre 25 °C et 35 °C, pour des dextranes à masse molaire plus élevée, et on utilisera des températures entre 35 °C et 50 °C, de préférence entre 35 °C et 45 °C pour des dextranes à masse molaire plus faible. La température de la réaction a ainsi une grande influence sur la masse molaire des produits formés par l'enzyme de la présente invention.

Par ailleurs, on utilisera de préférence des concentrations en saccharose élevées, notamment de 200 à 600 g.L$^{-1}$, de préférence entre 200 et 400 g.L$^{-1}$, pour des réactions conduites à des températures élevées, notamment supérieures ou égales à 45 °C.

La masse molaire d'un dextrane synthétisé peut être très précisément contrôlée par le choix de la température et de la concentration en saccharose, ce qui est particulièrement avantageux car ceci permet de fournir de manière précise une large gamme de dextranes en termes de masse molaire.

En effet, comme mis en évidence dans la partie expérimentale, il a ici été observé de manière surprenante une variation linéaire de la masse molaire des dextranes obtenus lorsqu'on fixe la température de la synthèse (ou la concentration en saccharose), et qu'on fait varier la concentration en saccharose (ou la température de la synthèse). Ce résultat très surprenant et, à la connaissance des inventeurs, jamais observé auparavant, est une caractéristique particulièrement avantageuse de l'invention en ce qu'elle permet d'obtenir, via des températures allant de 20 °C à 50 °C et des concentrations initiales en saccharose allant de 50 g.L$^{-1}$ à 600 g.L$^{-1}$, des dextranes de masse molaire allant de 2.10$^3$ à 40.10$^3$ g.mol$^{-1}$.

Selon un mode de réalisation particulier de l'invention selon lequel on utilise une dextrane-saccharase de séquence d'acides aminés SEQ ID NO : 2, les dextranes ont une masse molaire allant de 7.10$^3$ à 25.10$^3$ g.mol$^{-1}$.

Ainsi, à titre d'exemple, en fixant la température à 25 °C, le procédé de synthèse de dextranes selon l'invention permet d'obtenir, à partir d'une dextrane-saccharase de séquence d'acides aminés SEQ ID NO : 2, un dextrane de masse molaire moyenne de 25.10$^3$ g.mol$^{-1}$ à partir de 50 g.L$^{-1}$ de saccharose et un dextrane de masse molaire moyenne

de $8.10^3$ g.mol$^{-1}$ à partir de 400 g.L$^{-1}$ de saccharose. De la même façon, à une concentration initiale en saccharose fixée à 200 g.L$^{-1}$, il est possible d'obtenir un dextrane dont la masse molaire moyenne est de $17.10^3$ g.mol$^{-1}$ à 25 °C et un dextrane dont la masse molaire moyenne est de $8,5.10^3$ g.mol$^{-1}$ à 45 °C.

Un objet de la divulgation concerne donc un dextrane susceptible d'être obtenu par le procédé tel que précédemment décrit.

Typiquement, un dextrane selon la divulgation est caractérisé en ce qu'il présente 100% de liaisons glucosidiques $\alpha$-1,6, une masse molaire moyenne en poids Mw comprise entre $2.10^3$ et $40.10^3$ g.mol$^{-1}$, de préférence entre $6,5.10^3$ et $32.10^3$ g.mol$^{-1}$, de préférence encore entre $7.10^3$ et $25.10^3$ g.mol$^{-1}$ et un indice de dispersité Di inférieur à 1,5. Un mode de réalisation particulier concerne un tel dextrane susceptible d'être obtenu par le procédé conforme à l'invention et décrit tel que précédemment.

Ainsi, les dextranes conformes à la divulgation sont parfaitement linéaires, en ce qu'ils ne présentent aucune ramification, comme on peut le mettre en évidence avec une méthode de mesure par RMN du proton avec le spectromètre Bruker Avance (500 MHz) (cf. résultats expérimentaux). Par ailleurs, les dextranes conformes à la divulgation présentent typiquement une faible masse molaire moyenne ($2.10^3$ à $40.10^3$ g.mol$^{-1}$, de préférence entre $6,5.10^3$ et $32.10^3$ g.mol$^{-1}$, de préférence entre $7.10^3$ à $25.10^3$ g.mol$^{-1}$), déterminée par analyse HPSEC, et sont peu polydisperses. Ce résultat est très surprenant lorsqu'il est obtenu par une dextrane-saccharase selon l'invention, les glucane-saccharases de la famille GH-70 étant connues pour synthétiser généralement des $\alpha$-glucanes de très hautes masses molaires, de $10^6$ à $10^8$ Da (7-11).

Un mode de réalisation particulier de l'invention concerne également une dextrane-saccharase ayant été immobilisée.

Un objet de l'invention se rapporte donc à un complexe comprenant un support et une protéine à activité dextrane-saccharase selon l'invention, caractérisé en ce que ladite protéine à activité dextrane-saccharase a été immobilisée sur ledit support.

Par la suite, ledit complexe obtenu pourra parfois être appelé « protéine à activité dextrane-saccharase immobilisée » ou « dextrane-saccharase immobilisée ».

Comme précédemment mentionné, une dextrane-saccharase selon l'invention peut être immobilisée sur le support par des techniques connues de l'homme du métier, par exemple, par formation de liaisons ioniques et/ou hydrophobes entre le support et la protéine (adsorption), formation de liaisons covalentes entre le support et la protéine, inclusion de la protéine dans le support ou encapsulation de la protéine dans le support. Une dextrane-saccharase selon l'invention ayant été immobilisée sur un support présentera plusieurs avantages, dont ceux d'augmenter la stabilité de ladite dextrane-saccharase immobilisée vis-à-vis de la température, du pH etc., de permettre la réutilisation de la dite dextrane-saccharase lors de plusieurs synthèses successives, de permettre le développement de procédés semi-continus et/ou continus etc.

De préférence, une méthode d'immobilisation par adsorption ou par formation de liaisons covalentes entre le support et la protéine pourra être utilisée.

Un mode de réalisation particulier de l'invention concerne donc un complexe tel que précédemment défini, caractérisé en ce que ladite dextrane-saccharase a été immobilisée sur ledit support par formation de liaisons covalentes, ioniques et/ou hydrophobes entre le support et la protéine.

L'homme du métier saura déterminer le support le plus adapté suivant la méthode d'immobilisation utilisée, permettant notamment d'obtenir de bons rendements d'immobilisation ainsi qu'une activité de l'enzyme immobilisée élevée.

On peut par exemple utiliser comme support pour une méthode d'immobilisation par adsorption, du polyméthacrylate haute porosité avec comme groupement fonctionnel un groupement amino à espaceur court (par exemple Sprinbeads AA130® commercialisé par Sprin Technologies), du polyméthacrylate avec comme groupement fonctionnel un groupement octadécyl (par exemple Sprinbeads AO110® commercialisé par Sprin Technologies), du polystyrène - DVB (DiVinyl-Benzène) (par exemple Sprinbeads SN110® commercialisé par Sprin Technologies) ou du méthacrylate d'époxy avec comme groupement fonctionnel un groupement époxy (par exemple Purolite ECR8214® commercialisé par Purolite).

On peut par exemple utiliser comme support pour une méthode d'immobilisation par de liaisons ioniques entre le support et la protéine, du polyméthacrylate avec comme groupement fonctionnel un groupement amine quaternaire (par exemple Sepabeads ECQ1A® commercialisé par Resindion) ou du styrène avec comme groupement fonctionnel un groupement amine quaternaire (par exemple Purolite ECR1604® commercialisé par Purolite).

De préférence, une telle immobilisation est effectuée avec du méthacrylate d'époxy avec comme groupement fonctionnel un groupement époxy, notamment du Purolite ECR8214® commercialisé par Purolite.

De manière particulièrement avantageuse, la masse molaire d'un dextrane synthétisé à partir d'une dextrane-saccharase immobilisée selon l'invention peut, tout comme une dextrane-saccharase selon l'invention non immobilisée, être très précisément contrôlée par le choix de la température et de la concentration en saccharose. Ceci permet de fournir de manière précise une plus large gamme de dextranes en termes de masse molaire, une dextrane-saccharase immobilisée selon l'invention produisant avantageusement des dextranes de masses plus faibles que ceux produits par une dextrane-saccharase selon l'invention non immobilisée dans des conditions identiques (cf. résultats expérimentaux).

Ainsi, un mode de réalisation particulier de l'invention concerne un procédé de synthèse de dextranes, dans lequel :

- on fournit du saccharose dans un milieu de synthèse,
- on met en contact un complexe selon l'invention avec ledit saccharose dans ledit milieu de synthèse pour former des dextranes,
- on isole optionnellement les dextranes obtenus.

Un objet de la divulgation concerne également un dextrane susceptible d'être obtenu par ledit procédé de synthèse de dextranes.

De préférence, un dextrane ainsi obtenu est caractérisé en ce qu'il présente 100% de liaisons glucosidiques $\alpha$-1,6, une masse molaire moyenne en poids Mw comprise entre $3.10^3$ et $10.10^3$ g.mol$^{-1}$, de préférence entre $4,2.10^3$ et $7,3.10^3$ g.mol$^{-1}$, et un indice de dispersité Di inférieur à 1,5.

Typiquement, à une température fixée à 30 °C, une dextrane-saccharase de séquence d'acides aminés SEQ ID NO : 2 immobilisée sur support Purolite ECR8214® produit un dextrane de masse molaire de $7,3.10^3$ g.mol$^{-1}$ à partir de 50 g.L$^{-1}$ de saccharose et un dextrane de masse molaire moyenne de $4,2.10^3$ g.mol$^{-1}$ à partir de 450 g.L$^{-1}$ de saccharose.

L'invention concerne aussi la synthèse de gluco-oligosaccharides. Les gluco-oligosaccharides sont des oligosaccharides constitués d'un enchaînement d'unités glucosyle associées par des liaisons osidiques (par exemple en $\alpha$-1,6, $\alpha$-1,2, $\alpha$-1,3 et/ou $\alpha$-1,4). Les réactions d'accepteur réalisées consistent en un transfert de résidus glucosyle à partir de saccharose sur d'autres molécules acceptrices glucidiques ajoutées au milieu de synthèse.

Un objet de l'invention concerne ainsi un procédé de synthèse de gluco-oligosaccharides, dans lequel :

- on fournit du saccharose et au moins un accepteur glucidique dans un milieu de synthèse,
- on met en contact une dextrane-saccharase ou un complexe selon l'invention avec ledit saccharose et ledit au moins un accepteur glucidique dans ledit milieu de synthèse pour former des gluco-oligosaccharides,
- on isole optionnellement les gluco-oligosaccharides obtenus.

L'homme du métier sait quel accepteur glucidique utiliser en fonction du type de gluco-oligosaccharide qu'il souhaite obtenir.

Par exemple, ledit au moins un accepteur glucidique est choisi entre le glucose, le maltose, l'isomaltose, les maltooligosaccharides et les gluco-oligosaccharides, et de préférence, choisi entre le glucose, le maltose et/ou l'isomaltose.

Les mêmes conditions de synthèse que décrites précédemment (milieu de synthèse, température, pH, concentrations initiales en saccharose etc.) sont applicables au présent procédé de synthèse de gluco-oligosaccharides.

Un objet de la divulgation concerne également un gluco-oligosaccharide susceptible d'être obtenu par le procédé de synthèse de gluco-oligosaccharides précédemment décrit.

Selon un mode de réalisation particulier de l'invention, les gluco-oligosaccharides obtenus sont des isomaltooligosaccharides. On entend par « isomaltooligosaccharides » des oligomères de glucoses liés uniquement par des liaisons $\alpha$-1,6. De tels isomaltooligosaccharides peuvent être obtenus par un procédé de synthèse de gluco-oligosaccharides tel que défini précédemment, dans lequel ledit au moins un accepteur glucidique est par exemple le glucose ou l'isomaltose, de préférence le glucose.

Un gluco-oligosaccharide selon la divulgation a en général une masse molaire comprise entre $0,4.10^3$ et $5.10^3$ g.mol$^{-1}$, de préférence entre $0,7.10^3$ et $3,4.10^3$ g.mol$^{-1}$.

Typiquement, à une température fixée à 30 °C, le procédé de synthèse de gluco-oligosaccharides selon l'invention permet d'obtenir, à partir d'une protéine à activité dextrane-saccharase de séquence d'acides aminés SEQ ID NO : 2, un gluco-oligosaccharide de masse molaire moyenne de $6,8.10^2$ g.mol$^{-1}$ à partir de 315 g.L$^{-1}$ de glucose et de 60 g.L$^{-1}$ de saccharose et un gluco-oligosaccharide de masse molaire moyenne de $3,4.10^3$ g.mol$^{-1}$ à partir de 88 g.L$^{-1}$ de glucose et 110 g.L$^{-1}$ de saccharose.

Typiquement, à une température fixée à 30 °C, le procédé de synthèse de gluco-oligosaccharides selon l'invention permet d'obtenir, à partir d'une protéine à activité dextrane-saccharase de séquence d'acides aminés SEQ ID NO : 2 immobilisée sur support Purolite ECR8214 ®, un gluco-oligosaccharide de masse molaire moyenne de $7.10^2$ g.mol$^{-1}$ à partir de 333 g.L$^{-1}$ de glucose et de 166 g.L$^{-1}$ de saccharose et un gluco-oligosaccharide de masse molaire moyenne de $2,4.10^3$ g.mol$^{-1}$ à partir de 88 g.L$^{-1}$ de glucose et 110 g.L$^{-1}$ de saccharose.

L'invention concerne aussi la synthèse de gluco-conjugués. Les glucane-saccharases peuvent en effet être utilisées pour la synthèse de gluco-conjugués par réaction d'accepteur, en introduisant en plus du saccharose, une molécule hydroxylée dans le milieu de synthèse.

La glucosylation de molécules hydroxylées peut être intéressante pour la synthèse de nouveaux composés, ou pour une modification de propriétés physico-chimiques telles que la solubilité par exemple.

On entend par « molécule hydroxylée » tout type de molécule non glucidique contenant au moins un groupement hydroxyle libre. Typiquement, une molécule hydroxylée est un flavonoïde, polyol, acide aminé. On peut par exemple glycosyler des catéchines, polyphénols ou des alkyl-polyglucosides.

Un objet de l'invention concerne donc un procédé de synthèse de composés gluco-conjugués, dans lequel :

- on fournit du saccharose et au moins une molécule hydroxylée dans un milieu de synthèse,
- on met en contact une dextrane-saccharase ou un complexe selon l'invention avec ledit saccharose et ladite au moins une molécule hydroxylée dans ledit milieu de synthèse pour former des composés gluco-conjugués,
- on isole optionnellement les composés gluco-conjugués obtenus.

Les mêmes conditions de synthèse que décrites précédemment (milieu de synthèse, température, pH, concentrations initiales en saccharose etc.) sont applicables au présent procédé de synthèse de gluco-conjugués.

Un objet de la divulgation concerne un gluco-conjugué susceptible d'être obtenu par le procédé de synthèse de gluco-conjugués précédemment décrit.

Des produits selon la divulgation, notamment des dextranes, gluco-oligosaccharides ou gluco-conjugués conformes à la divulgation, pourront être utilisés dans tout type d'applications pour lesquelles leurs caractéristiques fonctionnelles, notamment leur taille et leur type de liaisons osidiques, sont adaptées.

Un dextrane, gluco-oligosaccharide ou gluco-conjugué selon la divulgation peut par exemple être utilisé dans des applications pharmaceutique, cosmétique ou alimentaire. On peut, pour les applications possibles, se référer à la publication Vettori et al. (12). De manière illustrative mais non limitative, un dextrane selon l'invention peut être utilisé comme support ou base (par exemple dans un vaccin ou dans une composition pharmaceutique) (13) comme agent nutraceutique (13,14), comme agent stabilisant (par exemple stabilisant d'un antigène dans un vaccin, ou stabilisant de protéines ou de produits lyophilisés), comme agent immuno-stimulant ou prébiotique (15), comme agent en prévention de la cristallisation des sucres, comme substitut de plasma sanguin, comme transporteur de fer dans le traitement des anémies importantes (14), comme anti-coagulant, comme extenseur du plasma sanguin, dans la prévention des chocs post-opératoires, dans le traitement des brûlures, et dans la réduction de risques de thrombose ou d'embolies, comme ingrédient actif ou excipient dans les solutions ophtalmiques, comme excipient lors d'une lyophilisation en tant que diluant et/ou modificateur de la température de collapse, comme cryoprotecteur, comme agent pour le stockage d'organes pour la transplantation.

Un objet de la divulgation concerne une composition pharmaceutique, cosmétique ou alimentaire comprenant un dextrane selon la divulgation, un gluco-oligosaccharide selon la divulgation ou un gluco-conjugué selon la divulgation à titre de principe actif ou à titre d'excipient acceptable.

La divulgation concerne enfin des dérivés de dextranes de l'invention. On entend ici par « *dérivé de dextrane* », un dextrane de l'invention et subissant une ou plusieurs étapes de modifications chimiques connues, notamment choisies parmi une éthérification, une estérification ou une réticulation. On peut ainsi obtenir un dérivé de dextrane tel qu'un dextrane réticulé, un ester de dextrane, par exemple un ester inorganique de dextrane (phosphate de dextrane, sulfate de dextrane) ou un ester organique de dextrane, un éther de dextrane, par exemple un éther de dextrane non-ionique (dextrane alkylé, éther d'hydroxyalkyle ou éther hydroxyalkyle aryle de dextrine, éther de dextrane poly(éthylène-glycol)-alkyle) ou un éther de dextrane ionique (dextrane sulfopropylé, dextrane carboxyméthylé, dextrane 2-(Diéthylamino)éthyl). De telles techniques de modification chimiques, ainsi que les applications pour lesquelles les dextranes ainsi obtenus sont adaptés, sont bien connues de l'homme du métier. On peut par exemple se référer au document Heinze et al (16) et à la thèse de Ndegwa Henry Maina (17).

Un objet de l'invention se rapporte donc à un procédé de modification d'un dextrane conforme à l'invention, dans lequel le dextrane subit une ou plusieurs étapes de modification chimique.

De manière avantageuse, une étape de modification chimique est choisie parmi une éthérification, une estérification et une réticulation.

## FIGURES

**Figure** 1 : Représentation schématique de la structure primaire de DsrM (basée sur l'alignement protéique avec l'enzyme GFT180 de *Lactobacillus reuteri* 180). Cinq domaines sont distincts : domaine V en blanc, domaine IV en gris clair, domaine B en rayé, domaine A à bulles et domaine C en noir. Les motifs répétés YG selon la définition de Giffard et Jacques sont représentés par des motifs à carreaux.

**Figure 2** : Représentation schématique des structures primaires de DsrM, DsrM ΔPS ΔC-APY et DsrM Δ174-1317. Cinq domaines sont distincts : domaine V en blanc, domaine IV en gris clair, domaine B en rayé, domaine A à bulles et domaine C en noir. Les motifs répétés YG selon la définition de Giffard et Jacques sont représentés par des motifs à carreaux.

**Figure 3 :** Caractéristiques des supports d'immobilisation issus du commerce et utilisés pour l'immobilisation de l'enzyme DsrM ΔPS ΔC-APY.

**Figure 4** : Représentation graphique de l'activité enzymatique de la dextrane-saccharase tronquée DsrM ΔPS ΔC-APY en fonction de la température dans des conditions standards, à partir de 100g.L$^{-1}$ de saccharose dans 50 mM d'acétate de sodium, pH 5,75.

**Figure 5** : Représentation graphique de l'activité enzymatique de la dextrane-saccharase entière DsrM en fonction de la température dans des conditions standards, à partir de 100g.L$^{-1}$ de saccharose dans 50 mM d'acétate de sodium, pH 5,75.

**Figure 6** : Représentation graphique de l'activité enzymatique de la dextrane-saccharase tronquée DsrM ΔPS ΔC-APY en fonction du pH dans des conditions standards, à partir de 100g.L$^{-1}$ de saccharose, à 30 °C.

**Figure 7** : Représentation graphique de l'activité enzymatique de la dextrane-saccharase entière DsrM en fonction du pH dans des conditions standards, à partir de 100g.L$^{-1}$ de saccharose, à 30 °C.

**Figure 8 :** Représentation de la masse molaire des dextranes synthétisés par DsrM à 25 °C, pH 5,75 en fonction de la concentration initiale en saccharose.

**Figure 9** : Profil HPSEC-RI des produits synthétisés par DsrM (forme entière), après 24 h de réaction à partir de 100 g.L$^{-1}$ de saccharose à 30 °C, pH 5,75.

**Figure 10 :** Graphiques de droite : Contrôle de la masse moléculaire des dextranes synthétisés par l'enzyme DsrM ΔPS ΔC-APY à température fixée : 25°C (A), 30°C (B), 35°C (C), 40 °C (D), en faisant varier la concentration initiale du substrat (saccharose).
Graphiques de gauche : Profils HPSEC-RI des réactions enzymatiques à t=24h à partir de 50 g.L$^{-1}$, 100 g.L$^{-1}$, 200 g.L$^{-1}$, 300 g.L$^{-1}$ et 400 g.L$^{-1}$ de saccharose (respectivement, courbes du bas vers le haut) avec température fixée : 25°C (A), 30°C (B), 35°C (C), 40 °C (D).

**Figure 11** : Graphiques de droite : Contrôle de la masse moléculaire des dextranes synthétisés par l'enzyme DsrM ΔPS ΔC-APY à concentration initiale en saccharose fixée : 50 g.L$^{-1}$ (A), 100 g.L$^{-1}$(B), 200 g.L$^{-1}$(C), 300 g.L$^{-1}$ (D) et 400 g.L$^{-1}$(E), en faisant varier la température.
Graphiques de gauche : Profils HPSEC-RI des réactions enzymatique à t=24h à 25 °C, 30 °C, 35 °C, 40 °C et 45 °C (respectivement, courbes du bas vers le haut) avec concentration initiale en saccharose fixée : 50 g.L$^{-1}$ (A), 100 g.L$^{-1}$ (B), 200 g.L$^{-1}$ (C), 300 g.L$^{-1}$ (D) et 400 g.L$^{-1}$ (E).

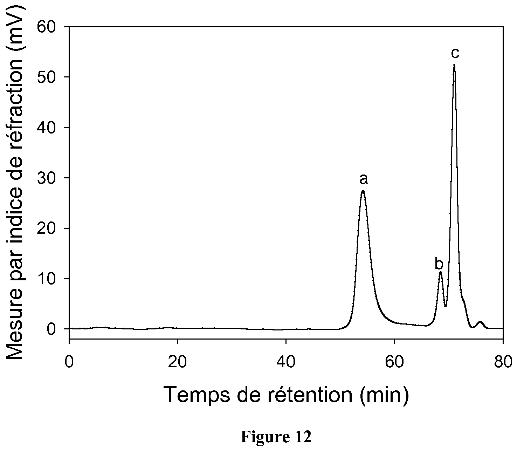

**Figure 12 :** Profil HPSEC-RI des dextranes synthétisés par DsrM ΔPS ΔC-APY en fin de réaction à partir de 100 g.L$^{-1}$ de saccharose à 30 °C, pH 5.75, avec a : polymère de masse moléculaire moyenne de 17,5 kDa, b : disaccharides (leucrose présent dans le mix de synthèse final) et c : mono-saccharides (fructose et glucose).

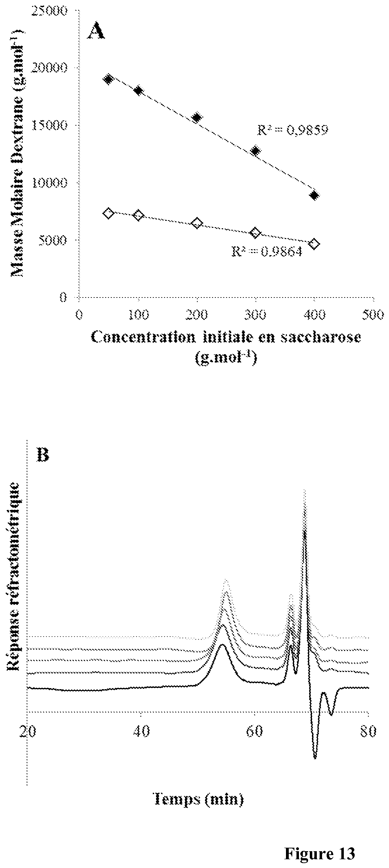

**Figure 13** : A : Comparaison de la masse molaire moyenne des dextranes synthétisés par l'enzyme DsrM ΔPS ΔC-APY libre (marqueur noir) et immobilisée sur support Purolite ECR8214® (marqueur blanc) à 30 °C et en fonction de concentrations initiales en saccharose croissantes (50 g.L$^{-1}$, 100 g.L$^{-1}$, 200 g.L$^{-1}$, 300 g.L$^{-1}$ et 400 g.L$^{-1}$)
B : Profils HPSEC-RI des réactions enzymatiques catalysées par DsrM ΔPS ΔC-APY immobilisée à t=24 h à partir de 50 g.L$^{-1}$, de 100 g.L$^{-1}$, de 200 g.L$^{-1}$, de 300 g.L$^{-1}$ et de 400 g.L$^{-1}$ de saccharose (respectivement, courbes du bas vers le haut).

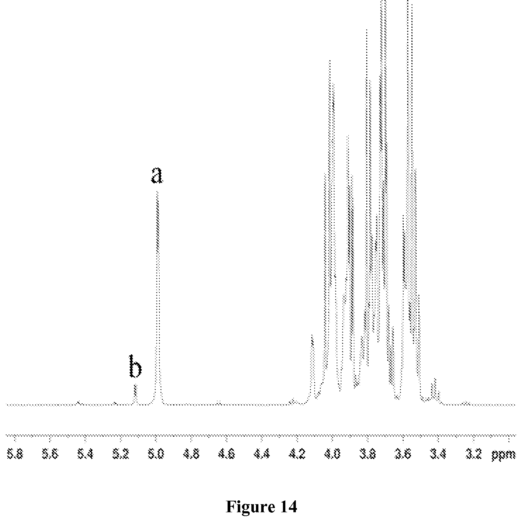

**Figure 14 :** Spectre H$^1$ RMN des produits synthétisés par DsrM après 24 h de réaction à partir de 100 g.L$^{-1}$ de saccharose à 30 °C, pH 5.75 avec a) liaisons α-1,6, b) leucrose.

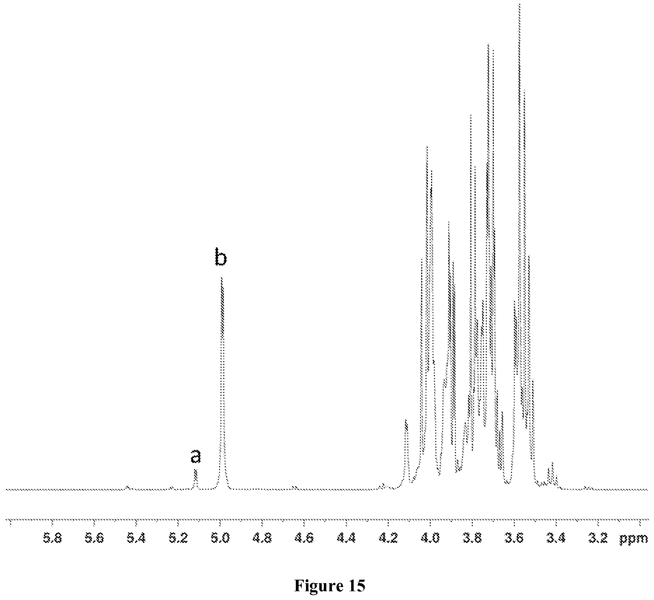

**Figure 15 :** Spectre H$^1$ RMN des produits synthétisés par DsrM ΔPS ΔC-APY en fin de réaction à partir de 100 g.L$^{-1}$ de saccharose à 30 °C, pH 5.75 avec a) leucrose, b) liaisons α-1,6.

**EXEMPLES**

**Exemple 1 : Identification du gène *dsrm* dans le génome *Leuconostoc citreum* NRRL B-1299 et analyse de la structure primaire de la protéine correspondante.**

Le gène *dsrm* a été identifié au sein du génome de *Leuconostoc citreum* NRRL B-1299 par blast nucléotidique contre une base de données constituée de séquences nucléotidiques de glucane-saccharases répertoriées dans la famille 70 des glycoside-hydrolases selon la classification CAZY (Carbohydrate Active enZYme database, www.cazy.org/GH70_all.html).

Le gène a été traduit en séquence protéique grâce au logiciel Transeq d'EMBOSS (www.ebi.ac.uk/Tools/st/emboss_transeq/).

Une séquence codant pour un peptide signal a été identifiée par le logiciel SignalP server 4.1 (www.cbs.dtu.dk/services/SignalP/).

Des alignements protéiques multiples (avec le logiciel d'alignement global, ClustalW2, disponible en ligne, www.ebi.ac.uk/Tools/msa/clustalw2/) avec d'autres glucane-saccharases caractérisées ont permis d'identifier les motifs conservés du coeur catalytique de DsrM, et de découper l'enzyme en différents domaines protéiques (A, B, C, IV et V).

Les différents pourcentages d'identité et de similarité entre séquences protéiques, indiqués dans la fiche préliminaire d'invention, ont été calculés avec l'outil BlastP (protein-protein Blast) du NCBI, disponible en ligne (blast.ncbi.nlm.nih.gov/Blast.cgi?PROGRAM=blastp&PAGE_TYPE=BlastSearch&LINK_L OC=blasthome) et en utilisant les paramètres par défaut proposés par le site.

**Exemple 2 : Clonage du gène *dsrm***

Le gène *dsrm* a été amplifié par PCR à partir de l'ADN génomique de *Leuconostoc citreum* NRRL B-1299 et en utilisant les deux amorces présentées dans le tableau 1.

*Tableau 1*

| Amorce | Séquence (5' à 3') |
|---|---|
| Amorce Forward | CACCATGAAAATAAAAGAAACAATTACCCGAAA (SEQ ID NO :3) |
| Amorce Reverse | AAGCTTGCAAAGCACGCTTATCAATC (SEQ ID NO :4) |

L'addition des 4 bases « CACC » en 5' de l'amorce forward (soulignées dans le tableau 1) a permis l'insertion correcte du fragment PCR dans le vecteur d'entrée pENTR/D/TOPO (Life Technologies), afin de procéder par la suite à un clonage utilisant la technologie Gateway. Un clone d'entrée positif (vecteur d'entrée contenant le fragment PCR dans le sens désiré) a été sélectionné et recombiné avec le vecteur de destination pET-55-DEST (Novagen) à l'aide de la LR clonase enzyme mix II (Life technologies). Les clones recombinants positifs ont été sélectionnés et analysés par restriction. L'absence de mutation au sein des plasmides a été confirmée par séquençage (GATC).

**Exemple 3: Clonage du gène *dsrm ⊿ps ⊿c-apy.***

Le gène *dsrm ⊿ps ⊿c-apy* été amplifié par PCR à partir du plasmide pET-55/DsrM précédemment construit à l'exemple 2 et en utilisant les deux amorces présentées dans le tableau 2.

*Tableau 2*

| Amorce | Séquence (5' à 3') |
|---|---|
| Amorce Forward | CACCCAAACGCCGGTTGGTACAACACAG (SEQ ID NO :5) |
| Amorce Reverse | TTTTGCCATCGTACCATCGTTATT (SEQ ID NO :6) |

L'addition des 4 bases « CACC » en 5' de l'amorce forward (soulignées dans le tableau 2) a permis l'insertion correcte du fragment PCR dans le vecteur d'entrée pENTR/D/TOPO (Life Technologies), afin de procéder par la suite à un clonage utilisant la technologie Gateway. Un clone d'entrée positif (vecteur d'entrée contenant le fragment PCR dans le sens désiré) a été sélectionné et recombiné avec le vecteur de destination pET-55-DEST (Novagen) à l'aide de la LR clonase enzyme mix II (Life technologies). Les clones recombinants positifs ont été sélectionnés et analysés par restriction. L'absence de mutation au sein des plasmides a été confirmée par séquençage (GATC).

**Exemple 4 : Expression hétérologue de *dsrm* et *dsrm Δps Δc-apy* chez *Escherichia coli***

Pour la production des enzymes recombinantes, des cellules d'*Escherichia coli* BL21 star DE3 ont été transformées avec les plasmides correspondants (pET-55/*dsrm* ou pET-55/ *dsrm Δps Δc-apy*) construits selon les exemples 2 et 3. 300 μL du mix de transformation ont été ensemencés dans 30 mL de milieu LB (Lysogeny Broth), supplémenté avec 100 μg.mL$^{-1}$ d'ampicilline, et mis à incuber sur la nuit à 37 °C afin de préparer une préculture.

Des cultures d'IL en milieu ZYM5052 modifié (1% glycérol, 0% glucose, 0.1% lactose, Studier, 2005) ont ainsi été ensemencées à une DO$_{600\,nm}$ initiale de 0,05 à partir de la pré-culture de la veille, puis mises à incuber 26 heures à 21 °C et à 150 rpm. En fin de fermentation, les milieux de culture sont centrifugés (15 min, 6500 rpm, 4 °C) et les culots sont concentrés à une DO de 80 dans 50 mM de tampon acétate de sodium, pH 5,75.

Afin d'obtenir l'enzyme recombinante (produite par *Escherichia coli* de manière intracellulaire), les cellules sont cassées aux ultrasons selon le protocole suivant : 5 cycles de 20 secondes à 30% de la puissance maximale de la sonde, à froid, espacés de 4 minutes de repos dans la glace. Le surnageant de sonication (contenant l'enzyme recombinante soluble) est ensuite récupéré après 30 minutes de centrifugation (10000 rpm, 10 °C) et conservé à 4°C.

**Exemple 5 : Méthode de détermination de l'activité enzymatique par la méthode du DNS.**

Une unité enzymatique glucane-saccharase représente la quantité d'enzyme qui libère une μmole de fructose par minute, à 30 °C, à partir de 100 g.L$^{-l}$ de saccharose dans 50mM acétate de sodium, pH 5,75.

L'activité est déterminée par mesure de la vitesse initiale de production des sucres réducteurs à l'aide de la méthode à l'acide dinitrosalicilique (DNS). Au cours d'une cinétique, 100 μL de milieu réactionnel sont prélevés et la réaction est stoppée par ajout d'un volume équivalent de DNS. Les échantillons sont ensuite chauffés 5 min à 95 °C, refroidis dans la glace, dilués au demi dans de l'eau, et l'absorbance est lue à 540 nm. Une gamme étalon de 0 à 2 g.L$^{-l}$ de fructose permet d'établir le lien entre valeur d'absorbance et concentration en sucres réducteurs.

**Exemple 6 : Immobilisation de la dextrane-saccharase tronquée DsrM ΔPS ΔC-APY**

Une immobilisation a été réalisée en faisant réagir des masses variant entre 0,1 g et 0,6 g de différents supports commerciaux avec 2,5 mL de surnageant de sonication contenant l'enzyme recombinante DsrM ΔPS ΔC-APY. Le pH de cette solution enzymatique est contrôlé et doit être compris entre 5,75 et 7,5. La réaction est menée à 4 °C sous agitation douce (100 rpm). L'immobilisation est stoppée après 16 heures de réaction par filtration et lavage avec trois volumes successifs (10 mL) de tampon acétate de sodium, pH 5,75 à 50 mM. L'enzyme immobilisée est conservée à 4 °C avant utilisation.

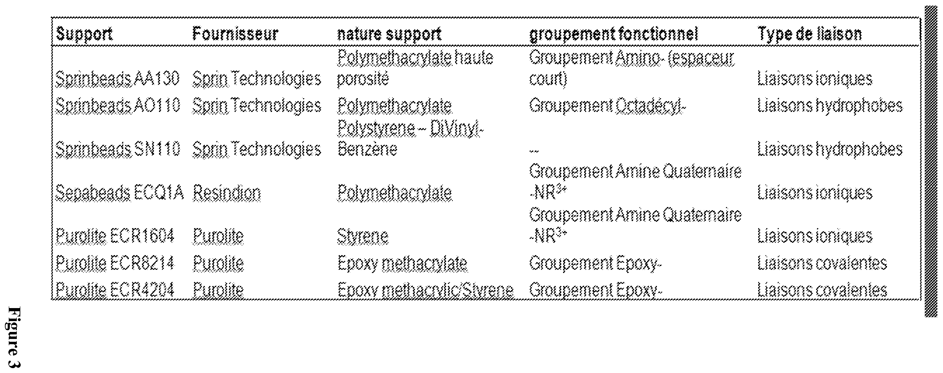

Les caractéristiques des supports testés sont données à la figure 3.

Par la suite, une telle enzyme immobilisée est appelée « DsrM ΔPS ΔC-APY immobilisée ».

Les différents supports d'immobilisation ont été testés pour leur capacité à fixer l'enzyme DsrM ΔPS ΔC-APY. Le crible a consisté à mettre en contact des masses de 250 mg de chaque support avec 2,5 mL de surnageant de sonication (activité libre initiale : 16,5 U/mL).

L'activité fixée après réaction et lavage sur les différents supports a été mesurée sur des aliquots issus de chaque lot d'enzyme produit et de masse contrôlée. Le volume réactionnel a également été adapté à l'activité de chaque lot, de plus gros volumes permettant la mesure des enzymes les plus actives. Les résultats du criblage sont présentés dans le tableau 3 suivant.

*Tableau 3*

| Support | Masse d'enzyme immobilisée en réaction (mg) | Volume réactionnel (ml) | Production de sucres réducteurs (μmol/min/réaction) | Activité (U/g) |
|---|---|---|---|---|
| Purolite ECR8214® | 39,5 | 3 | 1,03 | 77,94 |
| Sprinbeads AO110® | 35,8 | 3 | 0,93 | 77,77 |
| Sepabeads ECQ1A® | 36,7 | 3 | 0,43 | 35,34 |
| Purolite ECR1604® | 44,4 | 3 | 0,46 | 31,19 |

(suite)

| Support | Masse d'enzyme immobilisée en réaction (mg) | Volume réactionnel (ml) | Production de sucres réducteurs ($\mu$mol/min/réaction) | Activité (U/g) |
|---|---|---|---|---|
| Purolite ECR4204® | 56,5 | 1 | 1,71 | 30,27 |
| Sprinbeads AA130® | 54,4 | 1 | 1,32 | 24,26 |
| Sprinbeads SN110® | 43,4 | 3 | 0,32 | 21,88 |

Les différents supports en fonction de l'activité mesurée peuvent être regroupés en deux catégories :

- les 2 supports Purolite ECR8214® et Sprinbeads AO110® qui présentent les meilleures activités, de l'ordre de 78 U/g ;
- 5 supports ayant des activités moyennes mais bonnes, comprises entre 22 et 35 U/g ;

Seul le support Purolite ECR8214® a ensuite été utilisé afin d'optimiser l'immobilisation, notamment par une optimisation des rendements d'immobilisation, des activités fixées etc.

L'optimisation de l'immobilisation de DsrM ∆PS ∆C-APY sur Purolite ECR8214® a consisté à faire varier la masse de support entre 100 mg et 600 mg mise au contact d'une quantité d'enzyme libre fixe (2,5 mL de surnageant de sonication présentant une activité initiale de 45 U/mL).

Le calcul de trois différents rendements permettent de caractériser l'immobilisation :

- le rendement de fixation $R_{fixation}$, qui quantifie la part totale d'enzyme fixée sur les supports

$$R_{fixation}(\%) = \left(1 - \frac{Activité\ libre\ initiale}{Activité\ libre\ finale}\right) \times 100$$

- le rendement d'immobilisation $R_{immobilisation}$, qui quantifie l'enzyme immobilisée active en fonction de la quantité totale d'enzyme utilisée pour l'immobilisation

$$R_{immobilisation}(\%) = \frac{Activité\ immobilisée}{Activité\ libre\ introduite} \times 100$$

- l'efficacité d'immobilisation, qui quantifie la quantité d'enzyme immobilisée active en fonction de la quantité totale d'enzyme effectivement immobilisée

$$Efficacité\ (\%) = \frac{Activité\ immobilisée}{(Activité\ libre\ initiale - Activité\ libre\ finale)} \times 100$$

Les résultats d'immobilisation sont présentés dans le tableau 4 suivant.

*Tableau 4*

| masse pour immobilisation (mg) | Activité immobilisée U/g | Rendement de fixation % | Rendement d'immobilisation % | Efficacité % |
|---|---|---|---|---|
| 105,5 | 144,3 | 44 | 14 | 31 |
| 248,7 | 147,2 | 58 | 33 | 56 |

(suite)

| masse pour immobilisation (mg) | Activité immobilisée U/g | Rendement de fixation % | Rendement d'immobilisation % | Efficacité % |
|---|---|---|---|---|
| 355,6 | 146,0 | 80 | 46 | 58 |
| 456,7 | 148,2 | 92 | 60 | 66 |
| 604,0 | 132,3 | 97 | 71 | 73 |

La meilleure condition d'immobilisation est définie comme étant le résultat d'un compromis entre ces différents rendements et l'activité du biocatalyseur immobilisé obtenu.

Il est tout d'abord observé que l'utilisation d'une solution de DsrM ΔPS ΔC-APY libre d'activité plus élevée (45 U/mL vs 16U/mL utilisé lors du criblage des supports) permet d'augmenter significativement l'activité fixée sur le support. En effet, les activités mesurées sont augmentées d'un facteur 2 (cf tableau 4, activités de l'ordre de 140 U/mL).

Par ailleurs, des masses de support Purolite ECR8214® comprises entre 100 et 450 mg permettent l'obtention d'une activité fixée autour de 140 U/g. Sans vouloir être liés par une quelconque théorie, les inventeurs pensent que dans ces conditions, le support est totalement saturé de protéine. Cela est d'ailleurs confirmé par les rendements de fixation et d'immobilisation obtenus qui ont tendance à augmenter avec la masse de support utilisé.

Au-delà de 600 mg mis au contact avec 2,5 mL de surnageant de sonication, l'activité commence à diminuer (environ 132 U/g).

La meilleure condition d'immobilisation pour le support Purolite ECR8214® est donc la mise en contact d'une quantité comprise entre 450 et 600 mg de support, typiquement d'environ 500mg, avec 2,5 mL de surnageant de sonication. Ces conditions permettent l'obtention de la meilleure activité (environ 140 U/g) tout en limitant les pertes d'enzymes (rendements élevés).

Par la suite, « DsrM ΔPS ΔC-APY immobilisée » est une dextrane-saccharase DsrM ΔPS ΔC-APY immobilisée sur support Purolite ECR8214®.

**Exemple 7 : Détermination des rendements de production de la dextrane-saccharase entière DsrM et de la dextrane-saccharase tronquée DsrM ΔPS ΔC-APY**

Les rendements de production sont déterminés par chromatographie d'échange d'anion (HPAEC-PAD, High Performance Anion Exchange Chromatography with Pulsed Amperometric Detection) et par chromatographie d'exclusion de taille (HPSEC, High Performance Size Exclusion Chromatography).

Analyse HPAEC-PAD

Les sucres, glucose, fructose, leucrose et saccharose sont séparés sur colonne Dionex CarboPac PA-100 par un gradient d'acétate de sodium de 6 à 500 mM en 36 min, contenant 150 mM de soude. Des gammes étalons de 5, 10, 15 et 20 mg.kg$^{-1}$ de ces sucres sont réalisées et permettent leur quantification.

Les rendements de production, soit la part de glucose issu du saccharose incorporée dans la formation de glucose libre, de leucrose et de dextrane sont calculés comme suit :

$$\%G\ glucose = \frac{([Glucose]tf - [Glucose]t0) \times 342}{([Sucrose]t0 - [Sucrose]tf) \times 180} \times 100$$

$$\%G\ leucrose = \frac{([Leucrose]tf - [Leucrose]t0)}{([Sucrose]t0 - [Sucrose]tf)} \times 100$$

Analyse HPSEC

Les sucres sont séparés en fonction de leur taille sur deux colonnes de perméation de gel placées en série (Shodex OH Pack 805 et 802.5) dans un four dont la température est maintenue à 70 °C. Le débit de la phase mobile (0.45 M NaNO$_3$, 1% ethylene glycol) est de 0.3 mL.min$^{-1}$. Les échantillons sont dilués dans le même solvant que l'éluant à 10 g.L$^{-1}$ maximum de sucres totaux.

L'analyse des produits de la réaction par chromatographie d'exclusion de taille permet de calculer la part d'unités glucosyle issues du saccharose, utilisées dans la production de dextrane :

$$\%G\,dextrane = \frac{Aire_{dextrane}}{Aire_{saccharose} \times \frac{162}{342}}$$

Dextrane-saccharase entière DsrM

Il a ici été mis en évidence que l'enzyme DsrM est une très bonne polymérase. En effet, les analyses chromatographiques (HPAEC-PAD et HPSEC-RI) réalisées suite à une synthèse de dextrane à partir de 100 g.L$^{-1}$ de saccharose, à 30°C, pH 5,75, montrent que 81% des unités glucosyle issues du substrat sont utilisées pour la production de dextrane. Seuls 4% et 15% de ces unités sont incorporés dans la synthèse de glucose libre et de leucrose, respectivement.

Dextrane-saccharase tronquée DsrM ∆PS ∆C-APY

Il a ici été mis en évidence que la dextrane-saccharase tronquée DsrM ∆PS ∆C-APY est aussi une excellente polymérase. En effet, les analyses chromatographiques (HPAEC-PAD et HPSEC-RI) réalisées suite à une synthèse de dextrane à partir de 100 g.L$^{-1}$ de saccharose, à 30°C, pH 5.75, montrent que 85% des unités glucosyle issues du substrat sont utilisées pour la production du polymère. Seuls 3% et 12% de ces unités sont perdus par incorporation dans la synthèse de glucose libre et de leucrose, respectivement.

**Exemple 8 : Méthode de détermination de la masse molaire des dextranes par analyse HPSEC**

Une gamme étalon réalisée avec des dextranes commerciaux de 503000, 68400, 34100, 11300 g.mol$^{-1}$, ainsi que du maltopheptose, du saccharose et du fructose a permis de déterminer la masse molaire des dextranes synthétisés par DsrM, DsrM ∆PS ∆C-APY ou DsrM ∆PS ∆C-APY immobilisée.

**Exemple 9 : Détermination des conditions optimales de travail de la dextrane-saccharase entière DsrM et de la dextrane-saccharase tronquée DsrM ∆PS ∆C-APY**

Effet de la température

La valeur de la température optimale est déterminée en mesurant l'activité de l'extrait enzymatique brut, à différentes températures (entre 23 et 50 °C) à partir de 100 g.L$^{-1}$ de saccharose dans 50 mM de tampon acétate de sodium, pH 5,75.

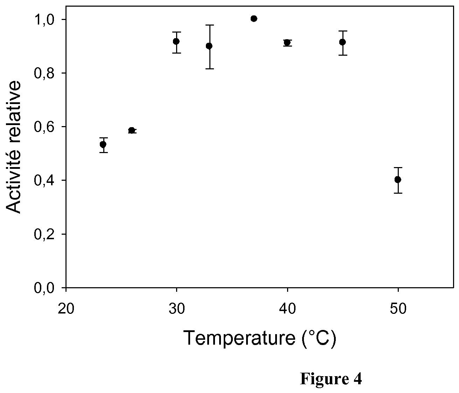

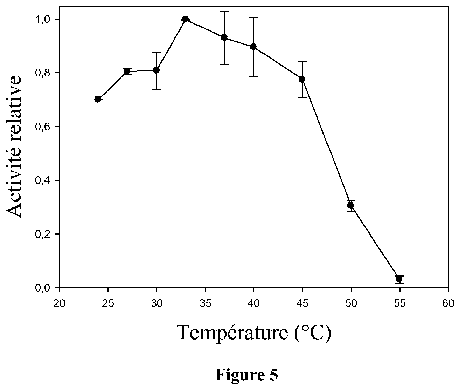

Comme visible aux figure 4 et 5, la dextrane-saccharase tronquée DsrM ∆PS ∆C-APY et la dextrane-saccharase DsrM ont une température optimale comprise 30 et 45°C, offrant la possibilité de travailler sur de larges gammes de températures, et notamment des températures élevées.

Effet du pH

L'effet du pH sur l'activité enzymatique de l'extrait brut est mesuré à 30 °C à partir de 100 g.L$^{-1}$ de saccharose, dans 50 mM de tampon citrate phosphate, pour des valeurs de pH comprises entre 3,5 et 8 (intervalle de 0,5).

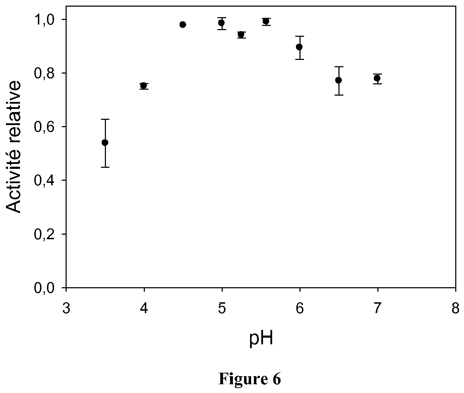

Comme visible à la figure 6, la dextrane-saccharase tronquée DsrM ∆PS ∆C-APY a un pH optimal compris entre 4,5 et 5,5.

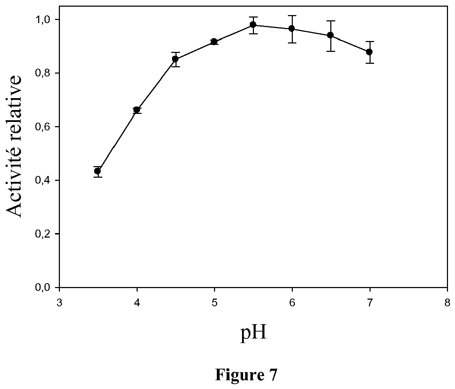

Comme visible à la figure 7, la dextrane-saccharase DsrM a un pH optimal compris entre 5 et 7.

**Exemple 10 : Production de dextranes de différentes masses molaires**

Des réactions enzymatiques avec DsrM, DsrM ∆PS ∆C-APY et DsrM ∆PS ∆C-APY immobilisée ont été mises en oeuvre à 1 U/mL, à partir de différentes concentrations initiales en saccharose (50, 100, 200, 300 et 400 g.L$^{-1}$) et éventuellement à différentes températures (25, 30, 35, 40 et 45 °C), dans du tampon acétate de sodium, 50 mM, pH 5.75.

Des prélèvements aux temps initiaux et finaux (24 h) ont été réalisés (la réaction enzymatique a été stoppée par chauffage à 95 °C pendant 5 minutes) et conservés à -20°C avant d'être analysés par HPAEC-PAD, comme expliqué à l'exemple 7, afin de contrôler les rendements de production, et par chromatographie d'exclusion de taille (HPSEC), comme expliqué à l'exemple 8, afin de déterminer la masse moléculaire des dextranes synthétisés.

En ce qui concerne les essais sur DsrM ΔPS ΔC-APY immobilisée, les milieux réactionnels sont centrifugés afin d'éliminer les résidus solides d'enzyme immobilisée.

Dextrane-saccharase entière DsrM

On observe une variation linéaire de la taille des produits en fonction de la concentration initiale en saccharose (fixation de la température).

Il est ainsi possible de contrôler la masse molaire du dextrane synthétisé par la forme entière de DsrM en faisant varier la concentration initiale en substrat, à température fixée.

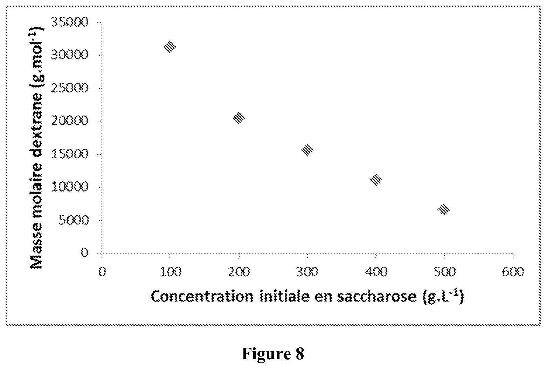

Ainsi, par exemple à 25 °C, il est possible d'obtenir un panel de dextranes dont la masse molaire moyenne varie entre $32.10^3$ et $6,5.10^3$ g.mol$^{-1}$ sur une gamme de concentration initiale en substrat allant de 100 à 500 g.L$^{-1}$ (Figure 8).

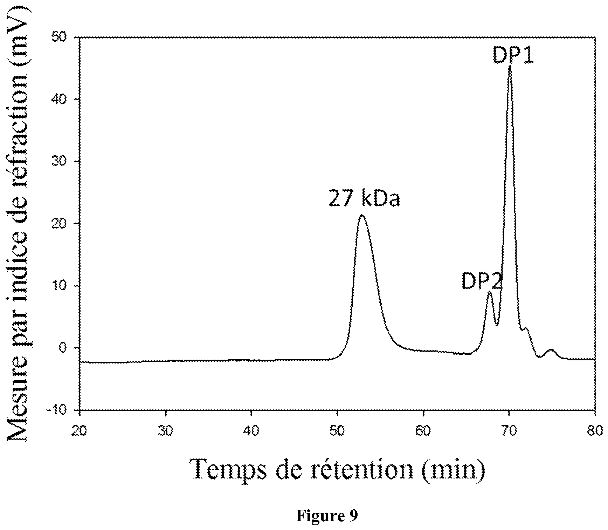

La figure 9 met en évidence qu'un dextrane, synthétisé à partir de 100 g.L$^{-1}$ de saccharose seul, à 30°C et pH 5.75, présente une faible masse molaire moyenne de $27.10^3$ Da, et est peu polydisperse.

Dextrane-saccharase tronquée DsrM ΔPS ΔC-APY

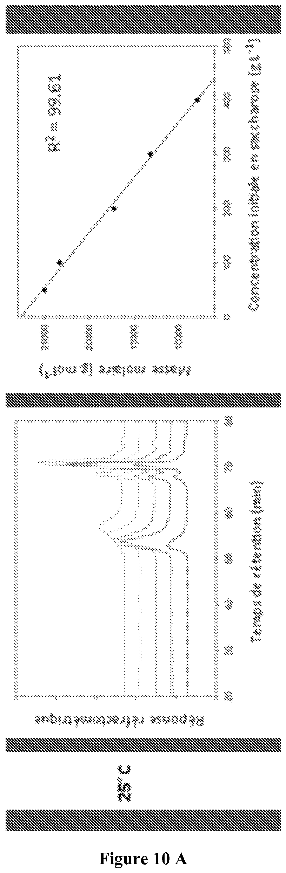

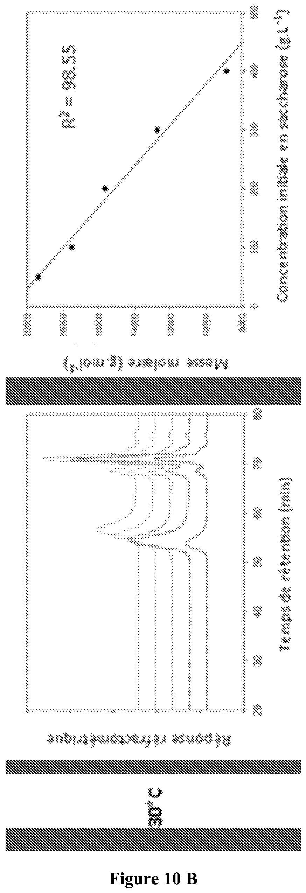

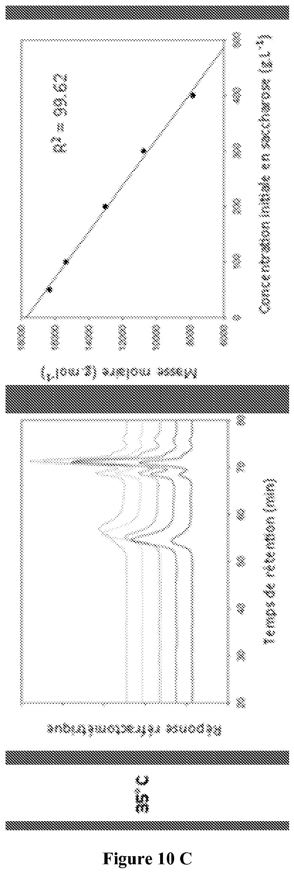

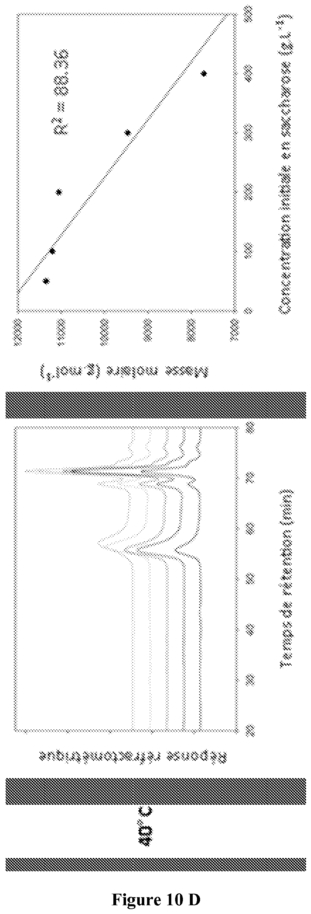

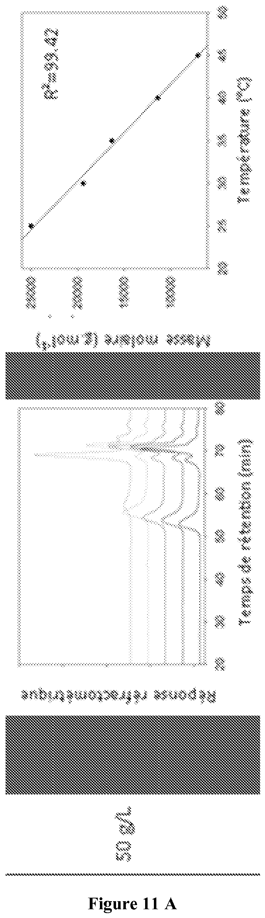

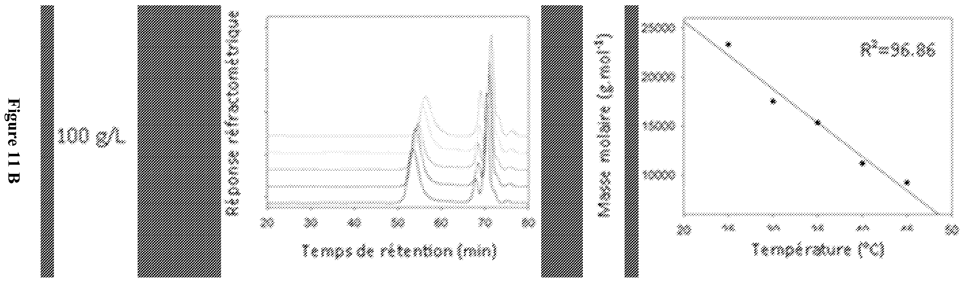

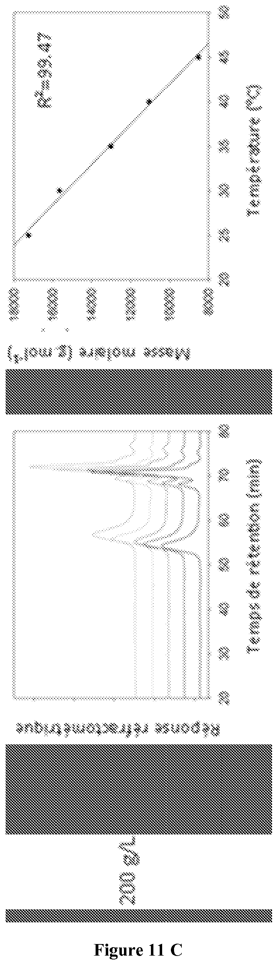

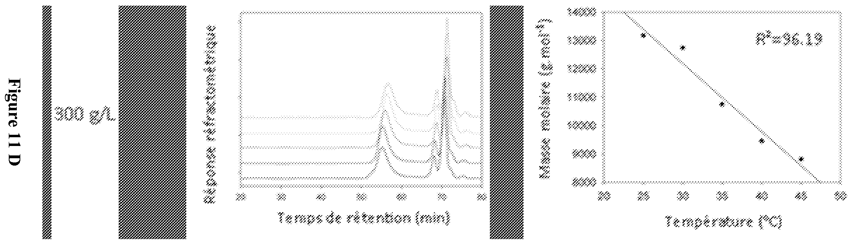

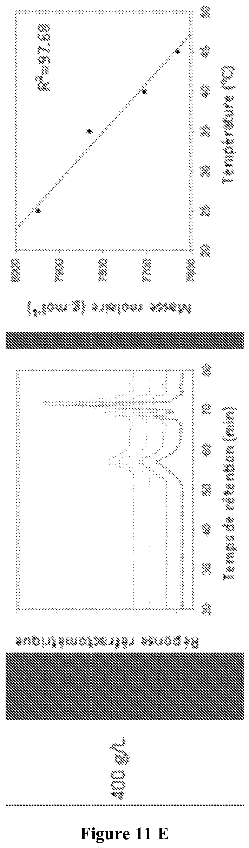

Comme visible aux figures 10 et 11, on observe une variation linéaire de la taille des produits en fonction de la température (fixation de la concentration initiale en saccharose) ou de la concentration initiale en saccharose (fixation de la température).

Il est ainsi également possible de contrôler de manière très précise la masse molaire du dextrane synthétisé par la dextrane-saccharase tronquée DsrM ΔPS ΔC-APY en faisant varier la concentration initiale en substrat, à température fixée (ou inverse).

Ainsi, à titre d'exemple, en fixant la température à 25 °C, la dextrane-saccharase tronquée DsrM ΔPS ΔC-APY produit un dextrane de masse molaire moyenne de $25.10^3$ g.mol$^{-1}$ à partir de 50 g.L$^{-1}$ de saccharose et un dextrane de masse molaire moyenne de $8.10^3$ g.mol$^{-1}$ à partir de 400 g.L$^{-1}$ de saccharose.

Par ailleurs, on remarque que les dextranes synthétisés sont peu polydisperses, à l'exception des polymères produits à partir de fortes concentrations en saccharose (400 g.L$^{-1}$).

On remarque aussi, qu'il est possible de balayer un plus large spectre de produits, en termes de masses moléculaires, à faibles températures ou à partir de petites concentrations en saccharose.

De la même façon, à une concentration initiale en saccharose fixée à 50 g.L$^{-1}$, il est possible d'obtenir des dextranes dont la masse molaire moyenne est comprise entre $25.10^3$ g.mol$^{-1}$ et $7.10^3$ g.mol$^{-1}$ sur une gamme de température allant de 25 à 45 °C.

De plus, les bilans de production varient peu en fonction des conditions expérimentales. Les analyses HPAEC-PAD effectuées montrent que pour toutes les réactions testées, plus de 81% des unités glucosyle issues du substrat sont utilisées pour la production du polymère lors de l'utilisation de dextrane-saccharase tronquée DsrM ΔPS ΔC-APY.

La figure 12 met en évidence qu'un dextrane, synthétisé à partir de 100 g.L$^{-1}$ de saccharose seul, à 30 °C et pH 5.75, présente une faible masse molaire moyenne de $17,5.10^3$ Da, et est peu polydisperse.

Dextrane-saccharase DsrM ΔPS ΔC-APY immobilisée

Comme visible à la figure 13, l'enzyme immobilisée conserve sa capacité à produire des dextranes de masses molaires différentes selon la concentration initiale en saccharose. La variation linéaire de la masse molaire des dextranes produits est également conservée.

Par ailleurs, la dispersité des dextranes obtenus est similaire entre les deux formes d'enzyme (dextrane-saccharase DsrM ΔPS ΔC-APY et dextrane-saccharase DsrM ΔPS ΔC-APY immobilisée) (figure 13).

Enfin, la dextrane-saccharase DsrM ΔPS ΔC-APY immobilisée sur support Purolite ECR8214® présente la particularité de produire des dextranes de masses molaires plus faibles que ceux produits par la dextrane-saccharase DsrM ΔPS ΔC-APY dans des conditions identiques. Par exemple, pour une concentration initiale de 100 g.L$^{-1}$ de saccharose, un dextrane de masse molaire $9,4.10^3$ g.mol$^{-1}$ est obtenu avec la dextrane-saccharase DsrM ΔPS ΔC-APY non immobilisée tandis qu'un dextrane de masse molaire $4,7.10^3$ g.mol$^{-1}$ est obtenu avec la dextrane-saccharase DsrM ΔPS ΔC-APY immobilisée (figure 13). L'immobilisation d'une dextrane-saccharase selon l'invention présente donc l'avantage d'élargir la gamme de dextranes produits.

**Exemple 11 : Analyse de la nature des liaisons des dextranes produits par la dextrane-saccharase entière DsrM et par la dextrane-saccharase tronquée DsrM ΔPS ΔC-APY**

Après lyophilisation, 20 mg de milieu réactionnel brut (après consommation totale du saccharose) sont dilués dans 0,5 mL d'eau deutérée et analysés par RMN du proton avec le spectromètre Bruker Avance (500 MHz). Les spectres sont ensuite traités et interprétés avec le logiciel TOPSPIN 3.0.

Dextrane-saccharase entière DsrM

Il a été mis en évidence par les analyses RMN que le produit synthétisé à partir de DsrM et 100 g.L$^{-1}$ de saccharose seul, à 30 °C dans 50 mM d'acétate de sodium, pH 5,75, est un polymère d'unités glucosyle liées exclusivement (100%) en α-1,6 (Figure 14).

Le produit synthétisé à partir de DsrM est donc un dextrane parfaitement linéaire, et DsrM est une dextrane-saccharase très spécifique de la polymérisation par liaisons osidiques de type α-1,6.

Dextrane-saccharase tronquée DsrM ΔPS ΔC-APY

Il a été mis en évidence par les analyses RMN que, tout comme pour la dextrane-saccharase DsrM, le produit synthétisé à partir de la forme tronquée DsrM ΔPS ΔC-APY et 100 g.L$^{-1}$ de saccharose seul, à 30 °C et pH 5,75 est un polymère d'unités glucosyle liées exclusivement (100%) en α-1,6 (Figure 15).

Le produit synthétisé à partir de la forme tronquée DsrM ΔPS ΔC-APY est donc également un dextrane parfaitement linéaire, et DsrM ΔPS ΔC-APY est une dextrane-saccharase très spécifique de la polymérisation par liaisons osidiques de type α-1,6.

**Exemple 12 : Production de gluco-oligosaccharides par réaction d'accepteur**

Des réactions d'accepteur ont été mises en oeuvre en utilisant 1 U.mL$^{-1}$ de DsrM ΔPS ΔC-APY et DsrM ΔPS ΔC-APY immobilisée, à partir de concentrations initiales en saccharose variant de 60 à 333 g.L$^{-1}$ et de concentrations en glucose (jouant le rôle d'accepteur glucidique) variant de 83 à 333 g.L$^{-1}$. Les synthèses ont eu lieu à une température de 30 °C et dans du tampon acétate de sodium, 50 mM, pH 5,75. Les réactions enzymatiques ont été stoppées par chauffage à 95 °C pendant 5 minutes après 24 heures.

Les différents échantillons ont ensuite été analysés par HPSEC comme expliqué à l'exemple 8 afin de déterminer la masse molaire moyenne des produits synthétisés.

Les résultats obtenus pour DsrM ΔPS ΔC-APY, i.e. la protéine à activité dextrane-saccharase ayant pour séquence d'acides aminés la séquence SEQ ID NO : 2, à partir de différentes concentrations en saccharose et en glucose accepteur sont présentés dans le tableau 5 suivant :

*Tableau 5*

| Ratio [Glucose]/ [Saccharose][a] | Masse Sèche Totale (g.L$^{-1}$) | Glucose (g.L$^{-1}$) | Saccharose (g.L$^{-1}$) | Masse molaire glucooligosaccharides (g.mol$^{-1}$) |
|---|---|---|---|---|
| 1,25 | 375 | 167 | 208 | $1,9.10^3$ |
| 2,31 | 375 | 113 | 262 | $2,7.10^3$ |
| 1,25 | 198 | 88 | 110 | $3,4.10^3$ |
| 0,50 | 500 | 333 | 167 | $0,8.10^3$ |
| 1,25 | 375 | 167 | 208 | $1,9.10^3$ |
| 0,19 | 375 | 315 | 60 | $0,7.10^3$ |
| 1,25 | 552 | 245 | 307 | $1,1.10^3$ |
| 2,00 | 500 | 167 | 333 | $1,6.10^3$ |
| 1,25 | 375 | 167 | 208 | $1,9.10^3$ |
| 0,50 | 250 | 167 | 83 | $1,6.10^3$ |
| 2,00 | 250 | 83 | 167 | $1,6.10^3$ |
| 1,25 | 375 | 167 | 208 | $1,9.10^3$ |

[a]Ratio calculé à partir de concentrations massiques

Les résultats obtenus pour DsrM ΔPS ΔC-APY immobilisée, i.e. la protéine à activité dextrane-saccharase ayant pour séquence d'acides aminés la séquence SEQ ID NO :2 immobilisée sur support ECR8214® comme décrit à l'exemple 6, à partir de différentes concentrations en saccharose et en glucose accepteur sont présentés dans le tableau 6 suivant :

*Tableau 6*

| Ratio [Glc]/ [Saccharose][a] | Masse Sèche Totale (g.L$^{-1}$) | [Glucose] (g.L$^{-1}$) | [Saccharose] (g.L$^{-1}$) | Masse molaire glucooligosaccharides (g.mol$^{-1}$) |
|---|---|---|---|---|
| 1,25 | 375 | 167 | 208 | $1,5.10^3$ |
| 2,31 | 375 | 113 | 262 | $2,0.10^3$ |
| 1,25 | 198 | 88 | 110 | $2,4.10^3$ |
| 0,50 | 500 | 333 | 167 | $0,7.10^3$ |
| 1,25 | 375 | 167 | 208 | $1,6.10^3$ |
| 0,19 | 375 | 315 | 60 | $0,7.10^3$ |
| 1,25 | 552 | 245 | 307 | $1,0.10^3$ |
| 2,00 | 500 | 167 | 333 | $1,4.10^3$ |
| 1,25 | 375 | 167 | 208 | $1,6.10^3$ |
| 1,25 | 375 | 167 | 208 | $1,5.10^3$ |
| 0,50 | 250 | 167 | 83 | $1,5.10^3$ |
| 2,00 | 250 | 83 | 167 | $2,3.10^3$ |
| 1,25 | 375 | 167 | 208 | $1,6.10^3$ |

[a]Ratio calculé à partir de concentrations massiques

Il a donc ici été démontré que, avec les conditions de synthèse mises en oeuvre, l'utilisation de dextrane-saccharases selon l'invention permet au moins de synthétiser des glucooligosaccharides de masse molaire moyenne allant de $0,7.10^3$ à $3,4.10^3$ g.mol-1.

**Exemple 13** :

Le dextrane commercial de 11 300 g/mol fourni par Sigma (ref D-9260 lot 74H0764) a été comparé au dextrane synthétisé par le procédé de l'invention à partir de saccharose 300 g/l dans du tampon acétate de sodium à pH 5.75 et une température de 30°C afin d'obtenir une masse molaire de 11 300 g/mol. Une analyse par chromatographie d'exclusion sur colonnes Shodex SB-805 et SB-802.5 en série montre que la population de dextranes obtenue grâce au procédé de l'invention est nettement moins polydisperse que le dextrane commercial.

**REFERENCES**

(1) Mage and Kabat, Immunochemical studies on dextrans; III. The specificities of rabbit antidextrans. Further findings on antidextrans with 1,2- and 1,6-specificities; 1963; Vol. 91, p. 634-640

(2) De Belder, Dextran; Amersham Biosciences; 2003

(3) Glucansucrases: Three-dimensional structures, reactions, mechanism, alpha-glucan analysis and their implications in biotechnology and food applications Lemhuis et al., Journal of Biotechnology, January 2013, vol 163, Issue 2, 250-272

(4) A method for détermination of invertase activity, Sumner & Howell, Journal of biological chemistry, 1935, vol 108, Issue 51

(5) Moulis C, Vaca Medina G, Suwannarangsee S, Monsan P, Remaud-Simeon M, Potocki-Veronese G (2008) One-step synthesis of isomalto-oligosaccharide syrups and dextrans of controlled size using engineered dextransucrase. Biocatal. Biotransformation. Taylor & Francis, pp 141-151

(6) Kim et al., Dextran molecular size and degree of branching as a function of sucrose concentration, pH, and temperature of reaction of Leuconostoc mesenteroides B-512FMCM dextransucrase; Carbohydrate Research 338 (2003) 1183-1189

(7) High-level production and purification of a fully active recombinant dextransucrase from Leuconostoc mesenteroides NRRL B-512F, Moulis et al, FEMS Microbiology Letters, August 2006, vol 261, Issue 2, 203-210

(8) Kothari et al, Structural Characterization of Enzymatically Synthesized Dextran and Oligosaccharides from Leuconostoc mesenteroides NRRL B-1426 Dextransucrase. ISSN 0006-2979, Biochemistry (Moscow), 2013, Vol. 78, No. 10, pp. 1164-1170.

(9) Amari et al, Characterization of a novel dextransucrase from Weissella confusa isolated from sourdough. Appl Microbiol Biotechnol (2013) 97:5413-5422

(10) Kralj et al, Glucan synthesis in the genus Lactobacillus: isolation and characterization of glucansucrase genes, enzymes and glucan products from six différent strains; Microbiology (2004), 150, 3681-3690.

(11) Striegel et al, An SEC/MALS study of alternan degradation during size-exclusion chromatographic analysis; Anal Bioanal Chem (2009) 394:1887-1893

(12) Dextran: effect of process parameters on production, purification and molecular weight and recent applications, Vettori et al., Diâlogos & Ciência, ISSN 1678-0493, no 31, septembre 2012

(13) GOULAS, A. K. et al. Synthesis of isomaltoligosaccharides and oligodextrans by the combined use of dextran-sucrase and dextranase. Enzyme Microb. Technol., 35, 2004. 327-338.

(14) Naessens et al., M. et al. Leuconostoc dextransucrase and dextran: production, properties and applications. J. Chem. Technol. Biotechnol., 80, 2005. 845-860.

(15) EGGLESTON AND COTE, G. L. Oligosaccharides in food and agriculture. Washington, DC: ACS symposium series 849, 2003. p. 1-15.

(16) Functional Polymers Based on Dextran, Thomas Heinze, Tim Liebert, Brigitte Heublein, Stephanie Hornig, Adv Polym Sci (2006) 205: 199-291, DOI 10.1007/12_100.

(17) Structure and macromolecular properties of Weissella confusa and Leuconostoc citreum dextrans with a potential application in sourdough, Ndegwa Henry Maina, 1st June 2012, University of Helsinki, Department of Food and Environmental Sciences, Chemistry and Biochemistry Division.

## Revendications

1. Protéine à activité dextrane-saccharase ayant pour séquence d'acides aminés la séquence SEQ ID NO : 1, ou comprenant au moins 80%, de préférence 85%, de préférence encore 90%, de préférence encore 95%, de préférence encore 98% d'identité sur les positions 563 à 1282 de la séquence d'acides aminés SEQ ID NO : 1, de préférence sur les positions 563 à 1282 et 1316 à 1433 de la séquence d'acides aminés SEQ ID NO : 1, de préférence encore sur les positions 563 à 1282, 1316 à 1433 et 174 à 421 de la séquence d'acides aminés SEQ ID NO : 1, de préférence encore sur les positions 563 à 1282, 1316 à 1433 et 42 à 421 de la séquence d'acides aminés SEQ ID NO : 1, l'activité dextrane saccharase permettant la synthèse de dextranes de masse molaire moyenne en poids $M_w$ comprise entre $2.10^3$ et $40.10^3$ g.mol$^{-1}$, et un indice de dispersité $D_i$ inférieur à 1,5, à des températures allant de 20 °C à 50 °C et des concentrations initiales en saccharose allant de 50 g.L$^{-1}$ à 600 g.L$^{-1}$.

2. Protéine à activité dextrane-saccharase ayant pour séquence d'acides aminés la séquence SEQ ID NO : 2, ou comprenant au moins 80%, de préférence 85%, de préférence encore 90%, de préférence encore 95%, de préférence encore 98% d'identité sur les positions 522 à 1241 sur la séquence d'acides aminés SEQ ID NO : 2, de préférence sur les positions 522 à 1241 et 1275 à 1392 sur la séquence d'acides aminés SEQ ID NO : 2, de préférence encore sur les positions 522 à 1241, 1275 à 1392 et 133 à 380 sur la séquence d'acides aminés SEQ ID NO : 2, de préférence encore sur les positions 522 à 1241, 1275 à 1392 et 1 à 380 sur la séquence d'acides aminés SEQ ID NO : 2, l'activité dextrane saccharase permettant la synthèse de dextranes de masse molaire moyenne en poids $M_w$ comprise entre $2.10^3$ et $40.10^3$ g.mol$^{-1}$, et un indice de dispersité $D_i$ inférieur à 1,5, à des températures allant de 20 °C à 50 °C et des concentrations initiales en saccharose allant de 50 g.L$^{-1}$ à 600 g.L$^{-1}$.

3. Protéine à activité dextrane-saccharase selon la revendication 2, **caractérisée en ce qu'**elle a pour séquence d'acides aminés la séquence SEQ ID NO : 2.

4. Complexe comprenant un support et une protéine à activité dextrane-saccharase telle que définie selon l'une quelconque des revendications 1 à 3, **caractérisé en ce que** ladite protéine à activité dextrane-saccharase a été immobilisée sur ledit support.

5. Procédé de synthèse de dextranes, dans lequel :

   - on fournit du saccharose dans un milieu de synthèse,
   - on met en contact une dextrane-saccharase telle que définie selon l'une quelconque des revendications 1 à 3 avec ledit saccharose dans ledit milieu de synthèse pour former des dextranes, et
   - on isole optionnellement les dextranes obtenus.

6. Procédé de synthèse de dextranes, dans lequel :

    - on fournit du saccharose dans un milieu de synthèse,
    - on met en contact un complexe tel que défini selon la revendication 4 avec ledit saccharose dans ledit milieu de synthèse pour former des dextranes, et
    - on isole optionnellement les dextranes obtenus.

7. Procédé de synthèse selon les revendications 5 ou 6, **caractérisé en ce que** la synthèse est conduite à une température comprise entre 20 °C et 50 °C, de préférence comprise entre 25 °C et 45 °C.

8. Procédé de synthèse selon l'une quelconque des revendications 5 à 7, **caractérisé en ce que** la concentration en saccharose dans le milieu de synthèse est comprise entre 50 et 600 g.L$^{-1}$, de préférence comprise entre 50 et 400 g.L$^{-1}$.

9. Procédé de synthèse selon l'une quelconque des revendications 5 à 8, **caractérisé en ce que** le pH lors de la réaction est compris entre 4 et 7, de préférence d'environ 5,75.

10. Procédé de synthèse de gluco-oligosaccharides dans lequel :

    - on fournit du saccharose et au moins un accepteur glucidique dans un milieu de synthèse,
    - on met en contact une dextrane-saccharase telle que définie selon l'une quelconque des revendications 1 à 3 ou un complexe tel que défini selon la revendication 4 avec ledit saccharose et ledit au moins un accepteur glucidique dans ledit milieu de synthèse pour former des gluco-oligosaccharides, et
    - on isole optionnellement les gluco-oligosaccharides obtenus.

11. Procédé de synthèse de composés gluco-conjugués, dans lequel :

    - on fournit du saccharose et au moins une molécule hydroxylée dans un milieu de synthèse,
    - on met en contact une dextrane-saccharase telle que définie selon l'une quelconque des revendications 1 à 3 ou un complexe tel que défini selon la revendication 4 avec ledit saccharose et ladite au moins une molécule hydroxylée dans ledit milieu de synthèse pour former des composés gluco-conjugués, et
    - on isole optionnellement les composés gluco-conjugués obtenus.

**Patentansprüche**

1. Protein mit Dextran-Saccharase-Aktivität, das als Aminosäuresequenz die Sequenz SEQ ID NR: 1 aufweist oder mindestens 80%, bevorzugt 85%, stärker bevorzugt 90%, stärker bevorzugt 95%, stärker bevorzugt 98% Identität an den Positionen 563 bis 1282 der Aminosäuresequenz SEQ ID NR: 1, bevorzugt an den Positionen 563 bis 1282 und 1316 bis 1433 der Aminosäuresequenz SEQ ID NR: 1, stärker bevorzugt an den Positionen 563 bis 1282, 1316 bis 1433 und 174 bis 421 der Aminosäuresequenz SEQ ID NR: 1, stärker bevorzugt an den Positionen 563 bis 1282, 1316 bis 1433 und 42 bis 421 der Aminosäuresequenz SEQ ID NR: 1, wobei die Dextran-Saccharase-Aktivität die Synthese von Dextranen mit durchschnittlichem Molekulargewicht $M_w$ zwischen $2.10^3$ und $40.10^3$ g.mol-1 ermöglicht und einen Dispersionsindex $D_i$ kleiner 1,5, bei Temperaturen von 20° C bis 50° C und initialen Saccharose-Konzentrationen von 50 g.L$^{-1}$ bis 600 g.L$^{-1}$.

2. Protein mit Dextran-Saccharase-Aktivität, das als Aminosäuresequenz die Sequenz SEQ ID NR: 2 aufweist oder mindestens 80%, bevorzugt 85%, stärker bevorzugt 90%, stärker bevorzugt 95%, stärker bevorzugt 98% Identität an den Positionen 522 bis 1241 der Aminosäuresequenz SEQ ID NR: 2, bevorzugt an den Positionen 522 bis 1241 und 1275 bis 1392 der Aminosäuresequenz SEQ ID NR: 2 umfasst, stärker bevorzugt an den Positionen 522 bis 1241, 1275 bis 1392 und 133 bis 380 der Aminosäuresequenz SEQ ID NR: 2, stärker bevorzugt an den Positionen 522 bis 1241, 1275 bis 1392 und 1 bis 380 der Aminosäuresequenz SEQ ID NR: 2, wobei die Dextran-Saccharase-Aktivität die Synthese von Dextranen mit durchschnittlichem Molekulargewicht $M_w$ zwischen $2.10^3$ und $40.10^3$ g.mol-1 ermöglicht und einen Dispersionsindex Di kleiner 1,5 bei Temperaturen von 20° C bis 50° C und initialen Saccharose-Konzentrationen von 50 g.L$^{-1}$ bis 600 g.L$^{-1}$.

3. Protein mit Dextran-Saccharase-Aktivität nach Anspruch 2,
**dadurch gekennzeichnet, dass** es als Aminosäuresequenz die Sequenz SEQ ID NR: 2 aufweist.

**4.** Komplex, welcher einen Träger und ein Protein mit Dextran-Saccharase-Aktivität nach einem der Ansprüche 1 bis 3 umfasst, **dadurch gekennzeichnet, dass** das Protein mit Dextran-Saccharase-Aktivität auf dem Träger immobilisiert wurde.

**5.** Verfahren zur Synthese von Dextranen, bei welchem:

- Saccharose in einem Synthesemedium bereitgestellt wird,
- eine Dextran-Saccharase nach einem der Ansprüche 1 bis 3 mit der Saccharose in dem Synthesemedium in Kontakt gebracht wird, um Dextrane zu bilden, und
- die erhaltenen Dextrane gegebenenfalls isoliert werden.

**6.** Verfahren zur Synthese von Dextranen, bei welchem:

- Saccharose in einem Synthesemedium bereitgestellt wird,
- ein Komplex nach Anspruch 4 mit der Saccharose in dem Synthesemedium in Kontakt gebracht wird, um Dextrane zu bilden, und
- die erhaltenen Dextrane gegebenenfalls isoliert werden.

**7.** Syntheseverfahren nach Anspruch 5 oder 6, **dadurch gekennzeichnet, dass** die Synthese bei einer Temperatur von 20° C bis 50° C, bevorzugt von 25° C bis 45° C, durchgeführt wird.

**8.** Syntheseverfahren nach einem der Ansprüche 5 bis 7, **dadurch gekennzeichnet, dass** die Saccharose-Konzentration im Synthesemedium zwischen 50 und 600 g.L$^{-1}$, bevorzugt zwischen 50 und 400 g.L$^{-1}$ liegt.

**9.** Syntheseverfahren nach einem der Ansprüche 5 bis 8, **dadurch gekennzeichnet, dass** der pH-Wert während der Reaktion zwischen 4 und 7, bevorzugt bei etwa 5,75, liegt.

**10.** Verfahren zur Synthese von Gluco-Oligosacchariden, bei welchem:

- Saccharose und mindestens ein Kohlenhydratakzeptor in einem Synthesemedium bereitgestellt werden,
- eine Dextran-Saccharase nach einem der Ansprüche 1 bis 3 oder ein Komplex nach Anspruch 4 mit der Saccharose und dem mindestens einen Kohlenhydratakzeptor in dem Synthesemedium zur Bildung von Gluco-Oligosacchariden in Kontakt gebracht wird, und
- die erhaltenen Gluco-Oligosaccharide gegebenenfalls isoliert werden.

**11.** Verfahren zur Synthese von glukokonjugierten Verbindungen, bei welchem:

- Saccharose und mindestens ein hydroxyliertes Molekül in einem Synthesemedium bereitgestellt werden,
- eine Dextran-Saccharase nach einem der Ansprüche 1 bis 3 oder ein Komplex nach Anspruch 4 mit der Saccharose und dem mindestens einen hydroxylierten Molekül in dem Synthesemedium zur Bildung von glukokonjugierten Verbindungen in Kontakt gebracht werden, und
- die erhaltenen glukokonjugierten Verbindungen gegebenenfalls isoliert werden.

**Claims**

**1.** A protein with dextransucrase activity, having the sequence SEQ ID NO:1 as amino acid sequence or comprising at least 80%, preferably 85%, more preferably still 90%, more preferably still 95%, more preferably still 98% identity at positions 563 to 1282 of the amino acid sequence SEQ ID NO: 1, preferably at positions 563 to 1282 and 1316 to 1433 of the amino acid sequence SEQ ID NO:1, more preferably still at positions 563 to 1282, 1316 to 1433 and 174 to 421 of the amino acid sequence SEQ ID NO:1, more preferably still at positions 563 to 1282, 1316 to 1433 and 42 to 421 of the amino acid sequence SEQ ID NO:1, the dextransucrase activity allowing the synthesis of dextrans having a weight-average molar mass $M_w$ of between $2x10^3$ and $40x10^3$ g.mol$^{-1}$, and a polydispersity index $I_p$ of less than 1.5, via temperatures ranging from 20°C to 50°C and initial sucrose concentrations ranging from 50 g.l$^{-1}$ to 600 g.l$^{-1}$.

**2.** A protein with dextransucrase activity, having the sequence SEQ ID NO: 2 as amino acid sequence or comprising at least 80%, preferably 85%, more preferably still 90%, more preferably still 95%, more preferably still 98% identity at positions 522 to 1241 of the amino acid sequence SEQ ID NO: 2, preferably at positions 522 to 1241 and 1275 to 1392 of the amino acid sequence SEQ ID NO:2, more preferably still at positions 522 to 1241, 1275 to 1392 and 133 to 380 of the amino acid sequence SEQ ID NO:2, more preferably still at positions 522 to 1241, 1275 to 1392 and 1 to 380 of the amino acid sequence SEQ ID NO: 2, the dextransucrase activity allowing the synthesis of dextrans having a weight-average molar mass $M_w$ of between $2 \times 10^3$ and $40 \times 10^3$ g.mol$^{-1}$, and a polydispersity index $I_p$ of less than 1.5, via temperatures ranging from 20°C to 50°C and initial sucrose concentrations ranging from 50 g.l$^{-1}$ to 600 g.l$^{-1}$.

3. The protein with dextransucrase activity as claimed in claim 2, **characterized in that** it has the sequence SEQ ID NO: 2 as amino acid sequence.

4. A complex comprising a support and a protein with dextransucrase activity as defined in any one of claims 1 to 3, **characterized in that** said protein with dextransucrase activity has been immobilized on said support.

5. A process for synthesizing dextrans, in which:

   - sucrose is provided in a synthesis medium,
   - a dextransucrase as defined in any one of claims 1 to 3 is placed in contact with said sucrose in said synthesis medium, to form dextrans, and
   - the dextrans obtained are optionally isolated.

6. A process for synthesizing dextrans, in which:

   - sucrose is provided in a synthesis medium,
   - a complex as defined in claim 4 is placed in contact with said sucrose in said synthesis medium, to form dextrans, and
   - the dextrans obtained are optionally isolated.

7. The synthesis process as claimed in claim 5 or 6, **characterized in that** the synthesis is carried out at a temperature of between 20°C and 50°C, preferably of between 25°C and 45°C.

8. The synthesis process as claimed in any one of claims 5 to 7, **characterized in that** the sucrose concentration in the synthesis medium is between 50 and 600 g.l$^{-1}$, preferably between 50 and 400 g.l$^{-1}$.

9. The synthesis process as claimed in any one of claims 5 to 8, **characterized in that** the pH during the reaction is between 4 and 7, preferably approximately 5.75.

10. A process for synthesizing glucooligosaccharides, in which:

    - sucrose and at least one carbohydrate acceptor are provided in a synthesis medium,
    - a dextransucrase as defined in any one of claims 1 to 3 or a complex as defined in claim 4 is placed in contact with said sucrose and said at least one carbohydrate acceptor in said synthesis medium, to form glucooligosaccharides, and
    - the glucooligosaccharides obtained are optionally isolated.

11. A process for synthesizing glucoconjugate compounds, in which:

    - sucrose and at least one hydroxylated molecule are provided in a synthesis medium,
    - a dextransucrase as defined in any one of claims 1 to 3 or a complex as defined in claim 4 is placed in contact with said sucrose and said at least one hydroxylated molecule in said synthesis medium, to form glucoconjugate compounds, and
    - the glucoconjugate compounds obtained are optionally isolated.

**GTF-180**

**DsrM**

Figure 1

Figure 2

| Support | Fournisseur | nature support | groupement fonctionnel | Type de liaison |
|---|---|---|---|---|
| Sprinbeads AA130 | Sprin Technologies | Polymethacrylate haute porosité | Groupement Amino- (espaceur court) | Liaisons ioniques |
| Sprinbeads AO110 | Sprin Technologies | Polymethacrylate | Groupement Octadécyl- | Liaisons hydrophobes |
| Sprinbeads SN110 | Sprin Technologies | Polystyrene – DiVinyl- Benzène | -- | Liaisons hydrophobes |
| Sepabeads ECQ1A | Resindion | Polymethacrylate | Groupement Amine Quaternaire -NR$^{3+}$ | Liaisons ioniques |
| Purolite ECR1604 | Purolite | Styrene | Groupement Amine Quaternaire -NR$^{3+}$ | Liaisons ioniques |
| Purolite ECR8214 | Purolite | Epoxy methacrylate | Groupement Epoxy- | Liaisons covalentes |
| Purolite ECR4204 | Purolite | Epoxy methacrylic/Styrene | Groupement Epoxy- | Liaisons covalentes |

Figure 3

**Figure 4**

**Figure 5**

**Figure 6**

**Figure 7**

**Figure 8**

**Figure 9**

**Figure 10 A**

**Figure 10 B**

**Figure 10 C**

**Figure 10 D**

**Figure 11 A**

**Figure 11 B**

**Figure 11 C**

**Figure 11 D**

**Figure 11 E**

**Figure 12**

**Figure 13**

**Figure 14**

**Figure 15**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5229277 A
- EP 2365084 A

**Littérature non-brevet citée dans la description**

- **MAGE ; KABAT.** Immunochemical studies on dextrans; III. The specificities of rabbit antidextrans. *Further findings on antidextrans with 1,2- and 1,6-specificities,* 1963, vol. 91, 634-640
- **DE BELDER ; DEXTRAN.** *Amersham Biosciences,* 2003
- **LEMHUIS et al.** Glucansucrases: Three-dimensional structures, reactions, mechanism, alpha-glucan analysis and their implications in biotechnology and food applications. *Journal of Biotechnology,* Janvier 2013, vol. 163 (2), 250-272
- **SUMNER ; HOWELL.** A method for détermination of invertase activity. *Journal of biological chemistry,* 1935, vol. 108 (51
- Potocki-Veronese G (2008) One-step synthesis of isomalto-oligosaccharide syrups and dextrans of controlled size using engineered dextransucrase. **MOULIS C ; VACA MEDINA G ; SUWANNARANG-SEE S ; MONSAN P ; REMAUD-SIMEON M.** Biocatal. Biotransformation. Taylor & Francis, 141-151
- **KIM et al.** Dextran molecular size and degree of branching as a function of sucrose concentration, pH, and temperature of reaction of Leuconostoc mesenteroides B-512FMCM dextransucrase. *Carbohydrate Research,* 2003, vol. 338, 1183-1189
- **MOULIS et al.** High-level production and purification of a fully active recombinant dextransucrase from Leuconostoc mesenteroides NRRL B-512F. *FEMS Microbiology Letters,* Août 2006, vol. 261 (2), 203-210
- **KOTHARI et al.** Structural Characterization of Enzymatically Synthesized Dextran and Oligosaccharides from Leuconostoc mesenteroides NRRL B-1426 Dextransucrase. *Biochemistry (Moscow),* 2013, vol. 78 (10), ISSN 0006-2979, 1164-1170
- **AMARI et al.** Characterization of a novel dextransucrase from Weissella confusa isolated from sourdough. *Appl Microbiol Biotechnol,* 2013, vol. 97, 5413-5422
- **KRALJ et al.** Glucan synthesis in the genus Lactobacillus: isolation and characterization of glucansucrase genes, enzymes and glucan products from six différent strains. *Microbiology,* 2004, vol. 150, 3681-3690
- **STRIEGEL et al.** An SEC/MALS study of alternan degradation during size-exclusion chromatographic analysis. *Anal Bioanal Chem,* 2009, vol. 394, 1887-1893
- **VETTORI et al.** Dextran: effect of process parameters on production, purification and molecular weight and recent applications. *Diâlogos & Ciência,* Septembre 2012, ISSN 1678-0493
- **GOULAS, A. K. et al.** Synthesis of isomaltoligosaccharides and oligodextrans by the combined use of dextransucrase and dextranase. *Enzyme Microb. Technol.,* 2004, vol. 35, 327-338
- **NAESSENS et al.** Leuconostoc dextransucrase and dextran: production, properties and applications. *J. Chem. Technol. Biotechnol.,* 2005, vol. 80, 845-860
- **EGGLESTON AND COTE, G. L.** Oligosaccharides in food and agriculture. *ACS symposium series,* 2003, vol. 849, 1-15
- Functional Polymers Based on Dextran, Thomas Heinze, Tim Liebert, Brigitte Heublein, Stephanie Hornig. *Adv Polym Sci,* 2006, vol. 205, 199-291
- Structure and macromolecular properties of Weissella confusa and Leuconostoc citreum dextrans with a potential application in sourdough. **NDEGWA HENRY MAINA.** Chemistry and Biochemistry Division. University of Helsinki, Department of Food and Environmental Sciences, 01 Juin 2012

SEQUENCE LISTING

<110> Institut National de la Recherche Agronomique

<120> Protéine à activité dextrane-saccharase et applications

<130> BCT150203 QT

<160> 6

<170> PatentIn version 3.5

<210> 1
<211> 2065
<212> PRT
<213> Artificial Sequence

<220>
<223> Dextrane saccharase

<400> 1

```
Met Lys Ile Lys Glu Thr Ile Thr Arg Lys Lys Leu Tyr Lys Ser Gly
1               5                   10                  15

Lys Ser Trp Val Ala Ala Ala Thr Ala Phe Ala Val Met Gly Val Ser
            20                  25              30

Ala Val Thr Thr Val Ser Ala Asp Thr Gln Thr Pro Val Gly Thr Thr
            35                  40              45

Gln Ser Gln Gln Asp Leu Thr Gly Gln Thr Gly Gln Asp Lys Pro Thr
        50                  55                  60

Thr Lys Glu Val Ile Asp Lys Lys Glu Pro Val Pro Arg Val Ser Ala
65                  70                  75                  80

Gln Asn Ala Gly Asp Leu Ser Ala Asp Ala Lys Thr Thr Lys Ala Asp
                85                  90                  95

Asp Lys Gln Asp Thr Gln Pro Thr Asn Ala Gln Leu Pro Asp Gln Gly
            100                 105                 110

Asn Lys Gln Thr Asn Ser Asn Ser Asp Lys Gly Val Lys Glu Ser Thr
            115                 120                 125

Thr Ala Pro Val Lys Thr Thr Asp Val Pro Ser Lys Ser Val Ala Pro
        130                 135                 140

Glu Thr Asn Thr Ser Ile Asn Ala Ser Asp Ala Ile Ser Lys Ser Gln
145                 150                 155                 160

Glu Lys Gln Phe Glu Lys Ala Pro Asp Ser Val Pro Glu Thr Ile Thr
```

|     |     | 165 |     |     |     |     |     | 170 |     |     |     |     |     | 175 |     |
|-----|-----|-----|-----|-----|-----|-----|-----|-----|-----|-----|-----|-----|-----|-----|-----|

Gly Gly Arg Tyr Ser Leu Lys Asp Gly Tyr Tyr Val Tyr Leu Asp Lys
                180                 185                 190

Gln Gly Lys Gln Val Val Gly Pro Lys Asn Ile Asp Asn His Leu Gln
                195                 200                 205

Tyr Phe Asp Glu Thr Thr Gly Lys Gln Val Lys Gly Asp Phe Arg Ser
        210                 215                 220

Val Asn Gly Lys Arg Ile Tyr Phe Asn Ala Asn Leu Gly Tyr Ala Asp
225                 230                 235                 240

Asp Tyr Thr Thr Asp Val Ala Gly Lys Leu Val Gly Tyr Asp Ser Asn
                245                 250                 255

Gly Asn Gln Val Lys Ala Gly Tyr Val Thr Asn Ser Gln Gly Lys Thr
                260                 265                 270

Tyr Tyr Phe Asn Asn Gln Gly Glu Ala Ile Ile Gly Leu Lys Thr Asp
                275                 280                 285

Asn Asn Lys Thr Gln Tyr Phe Gly Pro Asp Gly Ala Gln Val Lys Gly
        290                 295                 300

Ala Phe Gln Gln Val Asn Gly Lys Asn Ile Tyr Phe Asp Ala Gln Thr
305                 310                 315                 320

Gly Tyr Ala Arg Gln Asn Val Gly Phe Leu Asp Gly Thr Ala Lys Gly
                325                 330                 335

Phe Asp Glu Gln Gly Asn Gln Ile Lys Ser Gly Ile Ala Thr Asp Leu
                340                 345                 350

Ser Gly Asn Val Tyr Tyr Phe Asp Ala Ser Gly Lys Met Leu Thr Gly
        355                 360                 365

Val Gln Asn Ile Asp Gly Lys Lys Tyr Tyr Phe Asp Glu Gln Gly His
        370                 375                 380

Arg Arg Arg Asn Tyr Ala Gly Val Phe Asn Asn Glu Phe Ile Tyr Phe
385                 390                 395                 400

Gly Leu Asp Gly Val Gly Gln Ser Ala Ile Glu Tyr Gln Phe Glu Lys
                405                 410                 415

```
Gly Leu Thr Ser Gln Asn Ser Val Ala Thr Ser His Asn Ala Ala Lys
        420             425             430

Ser Tyr Asp Thr Lys Ser Phe Thr Asn Val Asp Gly Phe Leu Thr Ala
        435             440             445

Asn Ser Trp Tyr Arg Pro Thr Asp Ile Leu Arg Asn Gly Thr Lys Trp
        450             455             460

Glu Pro Ser Thr Glu Thr Asp Phe Arg Pro Leu Leu Met Thr Trp Trp
465             470             475             480

Pro Asp Lys Glu Val Gln Ala Asn Tyr Leu Asn Tyr Met Ser Ala Leu
                485             490             495

Gly Leu Gly Asp Gln Lys Ile Tyr Thr Gly Ala Ser Ser Gln Leu Asp
        500             505             510

Leu Asn Asn Ala Ala Leu Ile Val Gln Glu Ala Ile Glu Lys Lys Ile
        515             520             525

Ser Leu Glu Lys Ser Thr Lys Trp Leu Asp Asp Ser Ile Lys Ser Phe
        530             535             540

Ile Lys Ser Lys Arg Lys Asp Ile Gln Gly Asn Leu Val Asp Thr Asn
545             550             555             560

Pro Gly Trp Thr Ile Asp Ser Glu Thr Gly Ser Thr Asn His Leu Gln
                565             570             575

Asn Gly Ala Phe Ile Phe Thr Asn Ser Pro Leu Val Pro Glu Ala Asn
                580             585             590

Ala Ala Glu Gly Asn Arg Leu Ile Asn Arg Thr Pro Ser Gln Gln Thr
        595             600             605

Gly Asn His Ile Ser Tyr Ala Ser Gln Pro Tyr Ser Gly Asp Asp Trp
        610             615             620

Gly Tyr Glu Leu Leu Leu Gly Asn Asp Val Asp Asn Ser Asn Pro Ile
625             630             635             640

Val Gln Ala Glu Gln Leu Asn Trp Ile His Tyr Leu Met Asn Phe Gly
                645             650             655

Thr Ile Thr Ala Pro Gln Asp Pro Asp Ala His Leu Ala Asn Phe Asp
        660             665             670
```

Ser Ile Arg Ile Asp Ala Val Asp Asn Val Asp Ala Asp Leu Leu Gln
675 680 685

Ile Ala Gly Asp Tyr Phe Lys Ala Ala Tyr Gln Val Gly Glu Asn Asp
690 695 700

Lys Asn Ala Asn Gln His Ile His Ile Leu Glu Asp Trp Ser Pro Asn
705 710 715 720

Asp Val Trp Tyr Asn Gln Gln Val Asn Gly Asn Ser Gln Leu Thr Met
725 730 735

Asp Ala Thr Met Gln Asn Gln Leu Leu Ala Ser Leu Thr Arg Pro Ile
740 745 750

Thr Ser Arg Asp Ser Met Lys Ser Phe Thr Lys Asp Ala Leu Leu Val
755 760 765

His Arg Thr Ala Asp Asn Ser Tyr Asn Gln Ala Val Pro Asn Tyr Ser
770 775 780

Phe Ile Arg Ala His Asp Ser Glu Val Gln Thr Ile Ile Ala Lys Ile
785 790 795 800

Ile Ser Asp Lys His Pro Asp Leu Tyr Pro Thr Val Asp Lys Ala Leu
805 810 815

Leu Ala Lys Asp Ser Ala Leu Tyr Asp Glu Ala Phe Thr Glu Tyr Asn
820 825 830

Ala Asp Met Gln Lys Ile Ser Ser Gln Lys Gln Tyr Thr His Asn Asn
835 840 845

Met Pro Ser Ala Tyr Ala Ile Leu Leu Thr Asn Lys Asp Thr Val Pro
850 855 860

Arg Val Tyr Tyr Gly Asp Leu Phe Thr Asp Asn Gly Glu Tyr Met Ala
865 870 875 880

Asn Lys Thr Pro Tyr Tyr Asp Ala Ile Thr Ser Leu Leu Thr Ala Arg
885 890 895

Thr Lys Phe Val Ser Gly Gly Gln Ser Leu Ser Val Asp Lys Asn Asp
900 905 910

Val Leu Thr Ser Val Arg Tyr Gly Lys Gly Ala Leu Ser Ala Thr Asp
915 920 925

```
Asn Gly Ser Ser Asp Thr Arg Asn Gln Gly Ile Gly Val Ile Val Ser
    930                 935                 940

Asn Asn Pro Asn Leu Asp Leu Asn Asn Asp Lys Val Thr Leu Ser Met
945                 950                 955                 960

Gly Ile Ser His Ala His Gln Ala Tyr Arg Pro Leu Leu Leu Thr Asn
                965                 970                 975

Ser Gln Gly Ile Val Ala Tyr Ala Thr Asp Ser Glu Val Pro Gln Asn
                980                 985                 990

Leu Tyr Lys Thr Thr Asn Asp Lys  Gly Glu Leu Thr Phe  Asp Ala Ser
        995                 1000                1005

Glu Ile  Lys Gly Tyr Asp Thr  Val Gln Thr Ser Gly  Tyr Leu Ala
    1010                1015                1020

Val Trp  Val Pro Val Gly Ala  Ser Asp Glu Gln Asp  Ala Arg Thr
    1025                1030                1035

Ile Ala  Ser Thr Glu Lys Asn  Asn Gly Asn Ser Val  Tyr His Ser
    1040                1045                1050

Asn Ala  Ala Leu Asp Ser Gln  Leu Ile Tyr Glu Gly  Phe Ser Asn
    1055                1060                1065

Phe Gln  Thr Val Pro Ser Lys  Asn Ala Ser Ala Asp  Glu Tyr Ala
    1070                1075                1080

Asn Val  Ile Ile Ala Lys His  Ala Ala Asp Phe Asn  Lys Trp Gly
    1085                1090                1095

Val Thr  Ser Phe Gln Met Ala  Pro Gln Tyr Arg Ser  Ser Thr Asp
    1100                1105                1110

Gly Ser  Phe Leu Asp Ala Val  Asp Thr Val Gln Asn  Gly Tyr Ala
    1115                1120                1125

Phe Thr  Asp Arg Tyr Asp Leu  Gly Phe Asn Ala Ala  Asp Gly Ser
    1130                1135                1140

Lys Asn  Pro Thr Lys Tyr Gly  Thr Asp Glu Asp Leu  Arg Asn Ala
    1145                1150                1155

Ile Lys  Ser Leu His Ala Gln  Lys Thr Tyr Asp Gly  Ser Ser Ile
```

```
        1160                    1165                    1170


Gln Val  Met Ala Asp Phe Val  Pro Asp Gln Leu Tyr  Asn Met Pro
    1175                    1180                    1185


Leu Glu  Gln Ala Val Ser Val  Ile Arg Thr Asp Lys  Tyr Gly Val
    1190                    1195                    1200


Asn Ser  Glu Asn Pro Asp Ile  Gln Asn Ile Ile Tyr  Ala Ala Asn
    1205                    1210                    1215


Ile Lys  Ser Ser Gly Thr Asp  Tyr Gln Ser Ile Tyr  Gly Gly Lys
    1220                    1225                    1230


Tyr Leu  Ala Glu Leu Gln Lys  Asn Pro Leu Phe Lys  Ser Leu Phe
    1235                    1240                    1245


Asp Arg  Ile Gln Ile Ser Thr  Lys Lys Thr Ile Asp  Pro Asn Thr
    1250                    1255                    1260


Arg Ile  Thr Gln Trp Ser Ala  Lys Tyr Phe Asn Gly  Ser Asn Ile
    1265                    1270                    1275


Gln Gly  Lys Gly Ile Asn Tyr  Val Leu Lys Asp Trp  Ala Ser Asn
    1280                    1285                    1290


Lys Tyr  Phe Asn Val Ser Ser  Asn Asp Asp Met Tyr  Ser Arg Leu
    1295                    1300                    1305


Pro Lys  Gln Leu Met Asn Gln  Glu Ser Asn Thr Gly  Phe Ile Val
    1310                    1315                    1320


Asp Asp  Ile Gly Val Lys Tyr  Tyr Ser Ile Ser Gly  Tyr Gln Ala
    1325                    1330                    1335


Lys Asn  Thr Phe Val Glu Asp  Gly Asn Gly Glu Trp  Tyr Tyr Phe
    1340                    1345                    1350


Asp Asn  Asp Gly Tyr Met Val  Lys Ser Thr Glu Glu  Ser Gly Pro
    1355                    1360                    1365


Leu Arg  Thr Val Asn Ala Ser  Ser Lys Lys Tyr Tyr  Ile Leu Pro
    1370                    1375                    1380


Asn Gly  Val Glu Ile Arg Asn  Ser Phe Gly Gln Asp  Ile Gln Gly
    1385                    1390                    1395
```

```
Asn Thr  Tyr Tyr Phe Asp Ala  Arg Gly Glu Met Val  Thr Ser Gln
    1400              1405              1410

Tyr Ile  Ser Asp Asp Thr Gln  Asn Ile Tyr Tyr Phe  Asn Asn Asp
    1415              1420              1425

Gly Thr  Met Ala Lys Gly Leu  Ile Gln Leu Asn Thr  Asn Leu Gln
    1430              1435              1440

Tyr Phe  Gly Thr Asn Gly Ala  Gln Leu Lys Gly Ala  Tyr Val His
    1445              1450              1455

Asp Ile  Ser Ser Asp Lys Trp  Tyr Gln Phe Asp Ala  Gly Ser Gly
    1460              1465              1470

Asn Gly  Arg Gln Leu Thr Gln  Arg Pro Asp Asp Val  Asn Ala Asn
    1475              1480              1485

Asn Tyr  Ile Ser Ile Asp Ser  Ser Ser Asn Ile Gly  Val Asn Thr
    1490              1495              1500

Asp Tyr  Thr Ala Tyr Ile Thr  Ser Ser Leu Arg Glu  Asp Gly Leu
    1505              1510              1515

Phe Ala  Asn Ala Pro Tyr Gly  Val Val Thr Lys Asp  Gln Asn Gly
    1520              1525              1530

Asn Asp  Leu Lys Trp Gln Tyr  Ile Asn His Thr Lys  Gln Tyr Glu
    1535              1540              1545

Gly Gln  Gln Val Gln Val Thr  Arg Gln Tyr Thr Asp  Ser Lys Gly
    1550              1555              1560

Val Ser  Trp Asn Leu Ile Thr  Phe Ala Gly Gly Asp  Leu Gln Gly
    1565              1570              1575

Gln Lys  Leu Trp Val Asp Ser  Arg Ala Leu Thr Met  Thr Pro Phe
    1580              1585              1590

Lys Thr  Met Asn Gln Ile Ser  Phe Ile Ser Tyr Ala  Asn Arg Asn
    1595              1600              1605

Asp Gly  Leu Phe Leu Asn Ala  Pro Tyr Gln Val Lys  Gly Tyr Gln
    1610              1615              1620

Leu Ala  Gly Met Ser Asn Gln  Tyr Lys Gly Gln Gln  Val Thr Ile
    1625              1630              1635
```

27

```
Ala Gly Val Ala Asn Val Ser  Gly Lys Asp Trp Ser  Leu Ile Ser
    1640             1645              1650

Phe Asn Gly Thr Gln Tyr Trp  Ile Asp Ser Gln Ala  Leu Asn Thr
    1655             1660              1665

Asn Phe Thr His Asp Met Asn  Gln Lys Val Phe Val  Asn Thr Thr
    1670             1675              1680

Ser Asn Leu Asp Gly Leu Phe  Leu Asn Ala Pro Tyr  Arg Gln Pro
    1685             1690              1695

Gly Tyr Lys Leu Ala Gly Leu  Ala Lys Asn Tyr Asn  Asn Gln Thr
    1700             1705              1710

Val Thr Val Ser Gln Gln Tyr  Phe Asp Asp Gln Gly  Thr Val Trp
    1715             1720              1725

Ser Gln Val Val Leu Gly Gly  Gln Thr Val Trp Val  Asp Asn His
    1730             1735              1740

Ala Leu Ala Gln Met Gln Val  Arg Asp Thr Asn Gln  Gln Leu Tyr
    1745             1750              1755

Val Asn Ser Asn Gly Arg Asn  Asp Gly Leu Phe Leu  Asn Ala Pro
    1760             1765              1770

Tyr Arg Gly Gln Gly Ser Gln  Leu Ile Gly Met Thr  Ala Asp Tyr
    1775             1780              1785

Asn Gly Gln His Val Gln Val  Thr Lys Gln Gly Gln  Asp Ala Tyr
    1790             1795              1800

Gly Ala Gln Trp Arg Leu Ile  Thr Leu Asn Asn Gln  Gln Val Trp
    1805             1810              1815

Val Asp Ser Arg Ala Leu Ser  Thr Thr Ile Met Gln  Ala Met Asn
    1820             1825              1830

Asp Asp Met Tyr Val Asn Ser  Ser Gln Arg Thr Asp  Gly Leu Trp
    1835             1840              1845

Leu Asn Ala Pro Tyr Thr Met  Ser Gly Ala Lys Trp  Ala Gly Asp
    1850             1855              1860

Thr Arg Ser Ala Asn Gly Arg  Tyr Val His Ile Ser  Lys Ala Tyr
    1865             1870              1875
```

28

```
         Ser  Asn  Glu  Val  Gly  Asn  Thr   Tyr  Tyr  Leu  Thr  Asn   Leu  Asn  Gly
              1880                1885                1890


         Gln  Ser  Thr  Trp  Ile  Asp  Lys   Arg  Ala  Phe  Thr  Ala   Thr  Phe  Asp
              1895                1900                1905


         Gln  Val  Val  Ala  Leu  Asn  Ala   Thr  Ile  Val  Ala  Arg   Gln  Arg  Pro
              1910                1915                1920


         Asp  Gly  Met  Phe  Lys  Thr  Ala   Pro  Tyr  Gly  Glu  Ala   Gly  Ala  Gln
              1925                1930                1935


         Phe  Val  Asp  Tyr  Val  Thr  Asn   Tyr  Asn  Gln  Gln  Thr   Val  Pro  Val
              1940                1945                1950


         Thr  Lys  Gln  His  Ser  Asp  Ala   Gln  Gly  Asn  Gln  Trp   Tyr  Leu  Ala
              1955                1960                1965


         Thr  Val  Asn  Gly  Thr  Gln  Tyr   Trp  Ile  Asp  Gln  Arg   Ser  Phe  Ser
              1970                1975                1980


         Pro  Val  Val  Thr  Lys  Val  Val   Asp  Tyr  Gln  Ala  Lys   Ile  Val  Pro
              1985                1990                1995


         Arg  Thr  Thr  Arg  Asp  Gly  Val   Phe  Ser  Gly  Ala  Pro   Tyr  Gly  Glu
              2000                2005                2010


         Val  Asn  Ala  Lys  Leu  Val  Asn   Met  Ala  Thr  Ala  Tyr   Gln  Asn  Gln
              2015                2020                2025


         Val  Val  His  Ala  Thr  Gly  Glu   Tyr  Thr  Asn  Ala  Ser   Gly  Ile  Thr
              2030                2035                2040


         Trp  Ser  Gln  Phe  Ala  Leu  Ser   Gly  Gln  Glu  Asp  Lys   Leu  Trp  Ile
              2045                2050                2055


         Asp  Lys  Arg  Ala  Leu  Gln  Ala
              2060                2065
```

<210> 2
<211> 1392
<212> PRT
<213> Artificial Sequence

<220>
<223> Truncated dextrane saccharase

<400> 2

```
Gln Thr Pro Val Gly Thr Thr Gln Ser Gln Gln Asp Leu Thr Gly Gln
1               5               10              15

Thr Gly Gln Asp Lys Pro Thr Thr Lys Glu Val Ile Asp Lys Lys Glu
        20              25              30

Pro Val Pro Arg Val Ser Ala Gln Asn Ala Gly Asp Leu Ser Ala Asp
        35              40              45

Ala Lys Thr Thr Lys Ala Asp Asp Lys Gln Asp Thr Gln Pro Thr Asn
    50              55              60

Ala Gln Leu Pro Asp Gln Gly Asn Lys Gln Thr Asn Ser Asn Ser Asp
65              70              75              80

Lys Gly Val Lys Glu Ser Thr Thr Ala Pro Val Lys Thr Thr Asp Val
            85              90              95

Pro Ser Lys Ser Val Ala Pro Glu Thr Asn Thr Ser Ile Asn Ala Ser
        100             105             110

Asp Ala Ile Ser Lys Ser Gln Glu Lys Gln Phe Glu Lys Ala Pro Asp
        115             120             125

Ser Val Pro Glu Thr Ile Thr Gly Gly Arg Tyr Ser Leu Lys Asp Gly
        130             135             140

Tyr Tyr Val Tyr Leu Asp Lys Gln Gly Lys Gln Val Val Gly Pro Lys
145             150             155             160

Asn Ile Asp Asn His Leu Gln Tyr Phe Asp Glu Thr Thr Gly Lys Gln
                165             170             175

Val Lys Gly Asp Phe Arg Ser Val Asn Gly Lys Arg Ile Tyr Phe Asn
        180             185             190

Ala Asn Leu Gly Tyr Ala Asp Asp Tyr Thr Thr Asp Val Ala Gly Lys
        195             200             205

Leu Val Gly Tyr Asp Ser Asn Gly Asn Gln Val Lys Ala Gly Tyr Val
        210             215             220

Thr Asn Ser Gln Gly Lys Thr Tyr Tyr Phe Asn Asn Gln Gly Glu Ala
225             230             235             240

Ile Ile Gly Leu Lys Thr Asp Asn Asn Lys Thr Gln Tyr Phe Gly Pro
                245             250             255
```

```
Asp Gly Ala Gln Val Lys Gly Ala Phe Gln Gln Val Asn Gly Lys Asn
        260             265             270

Ile Tyr Phe Asp Ala Gln Thr Gly Tyr Ala Arg Gln Asn Val Gly Phe
        275             280             285

Leu Asp Gly Thr Ala Lys Gly Phe Asp Glu Gln Gly Asn Gln Ile Lys
        290             295             300

Ser Gly Ile Ala Thr Asp Leu Ser Gly Asn Val Tyr Tyr Phe Asp Ala
305             310             315             320

Ser Gly Lys Met Leu Thr Gly Val Gln Asn Ile Asp Gly Lys Lys Tyr
        325             330             335

Tyr Phe Asp Glu Gln Gly His Arg Arg Arg Asn Tyr Ala Gly Val Phe
        340             345             350

Asn Asn Glu Phe Ile Tyr Phe Gly Leu Asp Gly Val Gly Gln Ser Ala
        355             360             365

Ile Glu Tyr Gln Phe Glu Lys Gly Leu Thr Ser Gln Asn Ser Val Ala
        370             375             380

Thr Ser His Asn Ala Ala Lys Ser Tyr Asp Thr Lys Ser Phe Thr Asn
385             390             395             400

Val Asp Gly Phe Leu Thr Ala Asn Ser Trp Tyr Arg Pro Thr Asp Ile
        405             410             415

Leu Arg Asn Gly Thr Lys Trp Glu Pro Ser Thr Glu Thr Asp Phe Arg
        420             425             430

Pro Leu Leu Met Thr Trp Trp Pro Asp Lys Glu Val Gln Ala Asn Tyr
        435             440             445

Leu Asn Tyr Met Ser Ala Leu Gly Leu Gly Asp Gln Lys Ile Tyr Thr
        450             455             460

Gly Ala Ser Ser Gln Leu Asp Leu Asn Asn Ala Ala Leu Ile Val Gln
465             470             475             480

Glu Ala Ile Glu Lys Lys Ile Ser Leu Glu Lys Ser Thr Lys Trp Leu
        485             490             495

Asp Asp Ser Ile Lys Ser Phe Ile Lys Ser Lys Arg Lys Asp Ile Gln
```

                    500                        505                          510


        Gly Asn Leu Val Asp Thr Asn Pro Gly Trp Thr Ile Asp Ser Glu Thr
                    515                        520                   525


        Gly Ser Thr Asn His Leu Gln Asn Gly Ala Phe Ile Phe Thr Asn Ser
                    530                        535                   540


        Pro Leu Val Pro Glu Ala Asn Ala Ala Glu Gly Asn Arg Leu Ile Asn
        545                        550                   555                   560


        Arg Thr Pro Ser Gln Gln Thr Gly Asn His Ile Ser Tyr Ala Ser Gln
                              565                   570                   575


        Pro Tyr Ser Gly Asp Asp Trp Gly Tyr Glu Leu Leu Leu Gly Asn Asp
                          580                   585                   590


        Val Asp Asn Ser Asn Pro Ile Val Gln Ala Glu Gln Leu Asn Trp Ile
                    595                        600                   605


        His Tyr Leu Met Asn Phe Gly Thr Ile Thr Ala Pro Gln Asp Pro Asp
                    610                        615                   620


        Ala His Leu Ala Asn Phe Asp Ser Ile Arg Ile Asp Ala Val Asp Asn
        625                        630                   635                   640


        Val Asp Ala Asp Leu Leu Gln Ile Ala Gly Asp Tyr Phe Lys Ala Ala
                              645                   650                   655


        Tyr Gln Val Gly Glu Asn Asp Lys Asn Ala Asn Gln His Ile His Ile
                          660                   665                   670


        Leu Glu Asp Trp Ser Pro Asn Asp Val Trp Tyr Asn Gln Gln Val Asn
                          675                   680                   685


        Gly Asn Ser Gln Leu Thr Met Asp Ala Thr Met Gln Asn Gln Leu Leu
                    690                        695                   700


        Ala Ser Leu Thr Arg Pro Ile Thr Ser Arg Asp Ser Met Lys Ser Phe
        705                        710                   715                   720


        Thr Lys Asp Ala Leu Leu Val His Arg Thr Ala Asp Asn Ser Tyr Asn
                              725                   730                   735


        Gln Ala Val Pro Asn Tyr Ser Phe Ile Arg Ala His Asp Ser Glu Val
                          740                   745                   750

32

```
Gln Thr Ile Ile Ala Lys Ile Ile Ser Asp Lys His Pro Asp Leu Tyr
        755             760             765

Pro Thr Val Asp Lys Ala Leu Leu Ala Lys Asp Ser Ala Leu Tyr Asp
        770             775             780

Glu Ala Phe Thr Glu Tyr Asn Ala Asp Met Gln Lys Ile Ser Ser Gln
785             790             795             800

Lys Gln Tyr Thr His Asn Asn Met Pro Ser Ala Tyr Ala Ile Leu Leu
            805             810             815

Thr Asn Lys Asp Thr Val Pro Arg Val Tyr Tyr Gly Asp Leu Phe Thr
            820             825             830

Asp Asn Gly Glu Tyr Met Ala Asn Lys Thr Pro Tyr Tyr Asp Ala Ile
            835             840             845

Thr Ser Leu Leu Thr Ala Arg Thr Lys Phe Val Ser Gly Gly Gln Ser
        850             855             860

Leu Ser Val Asp Lys Asn Asp Val Leu Thr Ser Val Arg Tyr Gly Lys
865             870             875             880

Gly Ala Leu Ser Ala Thr Asp Asn Gly Ser Ser Asp Thr Arg Asn Gln
            885             890             895

Gly Ile Gly Val Ile Val Ser Asn Asn Pro Asn Leu Asp Leu Asn Asn
            900             905             910

Asp Lys Val Thr Leu Ser Met Gly Ile Ser His Ala His Gln Ala Tyr
            915             920             925

Arg Pro Leu Leu Leu Thr Asn Ser Gln Gly Ile Val Ala Tyr Ala Thr
        930             935             940

Asp Ser Glu Val Pro Gln Asn Leu Tyr Lys Thr Thr Asn Asp Lys Gly
945             950             955             960

Glu Leu Thr Phe Asp Ala Ser Glu Ile Lys Gly Tyr Asp Thr Val Gln
            965             970             975

Thr Ser Gly Tyr Leu Ala Val Trp Val Pro Val Gly Ala Ser Asp Glu
            980             985             990

Gln Asp Ala Arg Thr Ile Ala Ser  Thr Glu Lys Asn Asn  Gly Asn Ser
        995             1000            1005
```

```
Val Tyr His Ser Asn Ala Ala  Leu Asp Ser Gln Leu  Ile Tyr Glu
1010             1015             1020

Gly Phe Ser Asn Phe Gln Thr  Val Pro Ser Lys Asn  Ala Ser Ala
1025             1030             1035

Asp Glu Tyr Ala Asn Val Ile  Ile Ala Lys His Ala  Ala Asp Phe
1040             1045             1050

Asn Lys Trp Gly Val Thr Ser  Phe Gln Met Ala Pro  Gln Tyr Arg
1055             1060             1065

Ser Ser Thr Asp Gly Ser Phe  Leu Asp Ala Val Asp  Thr Val Gln
1070             1075             1080

Asn Gly Tyr Ala Phe Thr Asp  Arg Tyr Asp Leu Gly  Phe Asn Ala
1085             1090             1095

Ala Asp Gly Ser Lys Asn Pro  Thr Lys Tyr Gly Thr  Asp Glu Asp
1100             1105             1110

Leu Arg Asn Ala Ile Lys Ser  Leu His Ala Gln Lys  Thr Tyr Asp
1115             1120             1125

Gly Ser Ser Ile Gln Val Met  Ala Asp Phe Val Pro  Asp Gln Leu
1130             1135             1140

Tyr Asn Met Pro Leu Glu Gln  Ala Val Ser Val Ile  Arg Thr Asp
1145             1150             1155

Lys Tyr Gly Val Asn Ser Glu  Asn Pro Asp Ile Gln  Asn Ile Ile
1160             1165             1170

Tyr Ala Ala Asn Ile Lys Ser  Ser Gly Thr Asp Tyr  Gln Ser Ile
1175             1180             1185

Tyr Gly Gly Lys Tyr Leu Ala  Glu Leu Gln Lys Asn  Pro Leu Phe
1190             1195             1200

Lys Ser Leu Phe Asp Arg Ile  Gln Ile Ser Thr Lys  Lys Thr Ile
1205             1210             1215

Asp Pro Asn Thr Arg Ile Thr  Gln Trp Ser Ala Lys  Tyr Phe Asn
1220             1225             1230

Gly Ser Asn Ile Gln Gly Lys  Gly Ile Asn Tyr Val  Leu Lys Asp
1235             1240             1245
```

34

```
Trp Ala Ser Asn Lys Tyr Phe Asn Val Ser Ser Asn Asp Asp Met
    1250            1255            1260

Tyr Ser Arg Leu Pro Lys Gln Leu Met Asn Gln Glu Ser Asn Thr
    1265            1270            1275

Gly Phe Ile Val Asp Asp Ile Gly Val Lys Tyr Tyr Ser Ile Ser
    1280            1285            1290

Gly Tyr Gln Ala Lys Asn Thr Phe Val Glu Asp Gly Asn Gly Glu
    1295            1300            1305

Trp Tyr Tyr Phe Asp Asn Asp Gly Tyr Met Val Lys Ser Thr Glu
    1310            1315            1320

Glu Ser Gly Pro Leu Arg Thr Val Asn Ala Ser Ser Lys Lys Tyr
    1325            1330            1335

Tyr Ile Leu Pro Asn Gly Val Glu Ile Arg Asn Ser Phe Gly Gln
    1340            1345            1350

Asp Ile Gln Gly Asn Thr Tyr Tyr Phe Asp Ala Arg Gly Glu Met
    1355            1360            1365

Val Thr Ser Gln Tyr Ile Ser Asp Asp Thr Gln Asn Ile Tyr Tyr
    1370            1375            1380

Phe Asn Asn Asp Gly Thr Met Ala Lys
    1385            1390
```

<210> 3
<211> 33
<212> DNA
<213> Artificial Sequence

<220>
<223> primer

<400> 3
caccatgaaa ataaaagaaa caattacccg aaa        33

<210> 4
<211> 26
<212> DNA
<213> Artificial Sequence

<220>
<223> primer

<400> 4
aagcttgcaa agcacgctta tcaatc        26

<210> 5
<211> 28
<212> DNA
<213> Artificial Sequence

<220>
<223> primer

<400> 5
cacccaaacg ccggttggta caacacag          28

<210> 6
<211> 24
<212> DNA
<213> Artificial Sequence

<220>
<223> primer

<400> 6
ttttgccatc gtaccatcgt tatt          24